# EUROPEAN PATENT SPECIFICATION

(11) **EP 3 965 813 B1**
(45) Date of publication and mention of the grant of the patent: **20.11.2024**
(21) Application number: 20722624.2
(22) Date of filing: 07.05.2020
(51) Int. Cl.: A61K 39/12, C12Q 1/70, C12N 7/00

(54) **A NOVEL ORTHOBUNYAVIRUS IN HUMAN ENCEPHALITIS AND ITS DIAGNOSTIC AND THERAPEUTIC APPLICATIONS**
NEUARTIGER ORTHOBUNYAVIRUS IN DER HUMANEN ENCEPHALITIS UND DESSEN DIAGNOSTISCHE UND THERAPEUTISCHE ANWENDUNGEN
NOUVEL ORTHOBUNYAVIRUS DANS L'ENCÉPHALITE HUMAIN ET SES APPLICATIONS DIAGNOSTIQUES ET THÉRAPEUTIQUES

(30) Priority: 10.05.2019 EP 19305604
(43) Date of publication of application: 16.03.2022
(73) Proprietor: Institut Pasteur, 75724 Paris Cédex 15 (FR); Assistance Publique - Hôpitaux de Paris, 75004 Paris (FR); INSERM (Institut National de la Santé et de la Recherche Médicale), 75013 Paris (FR); Sorbonne Université, 75006 Paris (FR); Institut du Cerveau et de la Moelle Épinière-ICM, 75013 Paris (FR); Ecole Nationale Vétérinaire de Maisons Alfort, 94700 Maisons Alfort (FR); CENTRE NATIONAL DE LA RECHERCHE SCIENTIFIQUE -CNRS-, 75016 Paris (FR)
(72) Inventor: ELOIT, Marc, 75006 Paris (FR); PEROT, Philippe, 75014 Paris (FR); SEILHEAN, Danielle, 75019 Paris (FR); DUYCKAERTS, Charles, 94160 Saint Mandé (FR); CHRETIEN, Delphine, 93200 Saint Denis (FR)
(74) Representative: Plasseraud IP
(86) International application number: PCT/EP2020/062775
(87) International publication number: WO 2020/229315

(56) References cited:
- US-A1- 2013 323 210
- YADAV PRAGYA D ET AL: "A mini-review of Bunyaviruses recorded in India", INDIAN JOURNAL OF MEDICAL RESEARCH, vol. 145, May 2017 (2017-05-01), pages 600 - 609, XP002795364
- YADAV PRAGYA D ET AL: "Molecular characterization of Umbre virus (Bunyaviridae)", VIROLOGY JOURNAL, BIOMED CENTRAL, LONDON, GB, vol. 5, no. 1, 8 October 2008 (2008-10-08), pages 115, XP021045015, ISSN: 1743-422X
- JAN HELLERT ET AL: "Orthobunyavirus spike architecture and recognition by neutralizing antibodies", NATURE COMMUNICATIONS, vol. 10, no. 1, 20 February 2019 (2019-02-20), XP055637697, DOI: 10.1038/s41467-019-08832-8
- SUKHRALIA SHIVANI ET AL: "From dengue to Zika: the wide spread of mosquito-borne arboviruses", EUROPEAN JOURNAL OF CLINICAL MICROBIOLOGY & INFECTIOUS DISEASES, SPRINGER, WIESBADEN, DE, vol. 38, no. 1, 28 September 2018 (2018-09-28), pages 3 - 14, XP036664759, ISSN: 0934-9723, [retrieved on 20180928], DOI: 10.1007/S10096-018-3375-7

## Description

### FIELD OF THE INVENTION

The invention relates to methods of diagnosis or detection of Moissiacense virus, a novel orthobunyavirus causing human encephalitis, comprising determining the presence of at least one nucleic acid or protein of said virus or antibodies thereto, in a biological sample. The invention also relates to the various diagnostic agents derived from the viral nucleic acids or proteins, in particular nucleic acid primers and probes, antigens and antibodies, and their use for the diagnosis of Moissiacense virus infection and associated disease, in particular encephalitis. The invention further relates to antigens derived from the viral proteins as vaccine for the prevention of Moissiacense virus infection and associated disease, in particular encephalitis.

### BACKGROUND OF THE INVENTION

Human encephalitis is a potentially fatal disease of the central nervous system that is frequently associated with neurological sequelae. The incidence is about 1.5 - 7 cases / year / 100,000 inhabitants. In 20-50% of cases, encephalitis with known etiology is related to infection most often of viral origin (Glaser et al., Clin. Infect. Dis. Off. Publ. Infect. Dis. Soc. Am., 2006, 43, 1565-77). The most frequently implicated viruses belong to the *Herpesviridae* family (Herpes Simplex virus, Varicella Zoster virus and others), but also arboviruses depending on the geographical area, in particular Japanese encephalitis virus (JEV) in Asia and Tick-borne encephalitis virus (TBEV) in Europe and Northeast Asia.

Encephalitis is one of the most challenging syndromes as its etiology remains unknown in more than one third of the cases, even in countries benefiting from high-performance diagnostic technologies (Venkatesan et al., Clin. Infect. Dis. Off. Publ. Infect. Dis. Soc. Am., 2013, 57, 1114-28).

Indeed, new pathogens provoking encephalitis are recurrently identified (Mailles et al., Clin. Microbiol. Infect. Off. Publ. Eur. Soc. Clin. Microbiol. Infect. Dis., 2017, 23, 607-13). Diagnosis of encephalitis is also challenging because of the geographical spread of neurotropic arboviruses favored by climate change and human migration. In addition, this spread exposes human or animal populations devoid of specific immunity to these newly introduced arboviruses (Tabachnick WJ., Annu. Rev. Virol., 2016, 3, 125-45). A recent example is the Usutu virus, a flavivirus of the JEV complex responsible for human encephalitis, which has recently expanded in Europe (Ashraf et al., Viruses, 2015, 7, 219-38).

Etiological diagnosis of encephalitis is essential for appropriate management of patients (Nath A., JAMA Neurol., 2015, 72, 143-144). It is, however, particularly difficult as encephalitis can have autoimmune as well as viral origin (Ashraf et al., Viruses, 2015, 7, 219-38). Inaccurate diagnosis is a main issue as immunosuppressive treatments, in the case of undiagnosed infectious encephalitis, can be deleterious for the patient. In addition, the possibility of an infection underlying an autoimmune reaction renders the situation even more complex (Schein et al., Infection, 2017, 45, 545-9). In this context, identification of new pathogens responsible for encephalitis is critical for improving the scope of targeted diagnostic tests and diagnostic efficiency.

### SUMMARY OF THE INVENTION

The inventors have used agnostic metatranscriptomics procedures to analyze the brain tissue from two fatal cases of encephalitis for which a viral etiology had been suspected. This approach led to the identification and the characterization of Moissiacense virus, a novel orthobunyavirus group or species that includes Umbre virus a mosquito orthobunyavirus never described in humans before. This novel orthobunyavirus group or species is related to viruses of the Koongol group, a clade that had never previously been identified in vertebrates. It is of note that standard approach using PCR consensus assays targeting a broad range of orthobunyaviruses would have missed Moissiacense virus due to sequence variation. Viral sequences of the same clade were found in *Culex pipiens* mosquitoes captured in Southern France, which suggests that a novel arbovirus clade with neurotropic potential is present in Europe. Thus, Moissiacense virus should be added to serological tests and to targeted tests in routine diagnosis of brain tissue from encephalitis cases with unidentified etiology.

Therefore, in the various embodiments, the invention relates to methods of diagnosis or detection of Moissiacense virus, a novel orthobunyavirus causing human encephalitis, comprising determining the presence of at least one nucleic acid or protein of said virus or antibodies thereto, in a biological sample. The invention also relates to the various diagnostic agents derived from the viral nucleic acids or proteins, in particular nucleic acid primers and probes, antigens and antibodies, and their use for the diagnosis of Moissiacense virus infection and associated disease, in particular encephalitis. The invention further relates to antigens derived from the viral proteins as vaccine for the prevention of Moissiacense virus infection and associated disease, in particular encephalitis.

### DETAILED DESCRIPTION OF THE INVENTION

### Methods of diagnosis or detection of Moissiacense virus

The invention relates to methods of diagnosis or detection of Moissiacense virus, comprising determining the presence of at least one nucleic acid or protein of said virus or of antibodies against said virus, in a biological sample.

Moissiacense virus is a new neurotropic arbovirus belonging to orthobunayviruses, a genus of viruses within the family *Peribunyaviridae* in the order *Bunyavirales.* Orthobunyaviruses are spherical enveloped virus (80 nm to 120 nm in diameter) comprising a segmented Negative-stranded RNA linear genome encapsulated in a ribonucleocapsid. The L segment (about 6.9kb) encodes the viral RNA dependent RNA Polymerase or L Protein. The M segment (about 4.5kb) encodes a polyprotein which is cleaved by host protease into Gn, NSm and Gc proteins. The S segment (about 1kb) encodes the Nucleocapsid protein (N protein) and a non-structural protein (NSs Protein), by leaky scanning. The type species or prototype of orthobunyavirus is Bunyamwera (BUNV). As used herein, a bunyavirus refers to an orthobunyavirus. Unless otherwise specified herein, "Moissiacense virus", refers to the Moissiacense virus group or species which includes in particular, Moissiacense virus, a new virus identified by the inventors of the present application, as well as the known, Umbre and Little Sussex viruses. The Moissiacense virus group or species includes also the Mama virus, a new virus related to Moissiacense virus identified by the inventors of the present application. Based on the species demarcation criteria of the International Committee on Taxonomy of Viruses (ICTV) for orthobunyaviruses, Moissiacense virus, Marna virus, Umbre virus and Little Sussex virus would belong to the same species (>90% identical AA identity; ICTV report, 30 Nov 2018 (available from: https://talk.ictvonline.org/ictv-reports/ictv 9th report/). Therefore, taking the known Umbre virus as a reference, the Moissiacense virus and related Marna virus, newly identified by the inventors, are also designated as strains of Umbre virus or Umbre orthobunyavirus. The Moissiacense virus group is a subset of the Koongol virus group or clade which does not include Koongol virus. The sequences disclosed herein are those of the newly identified Moissiacense virus and related Mama virus, also named Umbre virus (or Umbre orthobunyavirus) strain Moissiacense and Umbre virus (or Umbre orthobunyavirus) strain Marna, respectively. All the sequences disclosed herein are in the 5' to 3' orientation. According to the standard practice in the field of bunyavirus (negative-sense single stranded RNA virus), viral nucleotide sequences are disclosed as the positive strand or sense strand coding sequences of the S, M, and L segments, in the DNA form. However, the present invention encompasses the RNA equivalent of the DNA sequences herein disclosed as well as their complement (i.e., reverse complement) sequences.

A sample or biological sample refers to any material obtained or derived from living or dead individual (human or animal) that may contain a Moissiacense virus or components thereof such as nucleic acids, proteins or fragments thereof, or antibodies against said Moissiacense virus appropriate for detection by the methods of the invention. Biological sample also includes any material derived or obtained from an arthropod vector that may contain a Moissiacense virus or components thereof such as nucleic acids, proteins or fragments thereof. The source of the sample may be any solid tissue as from fresh, frozen and/or preserved organ or tissue sample or biopsy or aspirate; any body fluid such as whole-blood or any blood constituent (serum, plasma), cerebral spinal fluid (CSF), peritoneal, pleural, synovial or amniotic fluid, urine, lymph and mucosal secretions. The sample may also be primary or cultured cells. The source of the sample can also be a whole vector specimen, a portion thereof or egg(s) thereof. The sample can be derived from any vector capable of transmitting Moissiacense virus, in particular a mosquitoe such as *Culex sp* or *Aedes sp.,* or others. Samples include also processed samples that have been treated to disrupt tissue or cell structure, thus releasing intracellular components into a solution which may further contain reagents (buffers, salts, detergents, enzymes and the like) which are used to prepare, using standard methods, a biological sample for analysis. In particular, processed samples include samples that have been treated by standard methods used for the isolation of nucleic acids or proteins from biological samples.

In some embodiments of the methods of the invention, the sample is a clinical sample (*i.e.,* a sample from living or dead individual (human or animal) suspected of having a Moissiacense virus). Preferably, the sample is brain biopsy, cerebral spinal fluid (CSF), whole-blood, plasma or serum. In some other embodiments, the biological sample is an arthropod vector specimen.

### Nucleic acid detection

In some aspects, the method of diagnosis or detection of Moissiacense virus comprises determining the presence of at least one nucleic acid of said virus in a sample.

In some embodiments, the viral nucleic acid is selected from the group consisting of:
- a first nucleic acid (S segment) comprising a sequence having at least 80% identity with SEQ ID NO: 1 and further including a coding sequence for a Nucleocapsid (N) protein, or its complement;
- a second nucleic acid (M segment) comprising a sequence having at least 80% identity with SEQ ID NO: 3 and further including a coding sequence for a polyprotein M, or its complement; and
- a third nucleic acid (L segment) comprising a sequence having at least 80% identity with SEQ ID NO: 5 and further including a coding sequence for a polymerase (L) protein, or its complement.

The first nucleic acid corresponds to the S segment which codes for the Nucleocapsid (N) protein or Nucleoprotein and for the nonstructural protein NSs. The N protein coding sequence or ORF is from positions 59 to 772 of SEQ ID NO: 1 and has the sequence SEQ ID NO: 73. The NSs protein coding sequence or ORF is from positions 96 to 335 of SEQ ID NO: 1 and has the sequence SEQ ID NO: 71.

The Nucleocapsid (N) protein refers to the protein of SEQ ID NO: 2 or a functional variant thereof. The protein of SEQ ID NO: 2 and its functional variants have the general properties of a bunyavirus nucleocapsid protein, taking as reference Bunyamwera (BUNV-N: GenBank NP_047213.1). The bunyavirus N protein is the major component of the viral nucleocapsid. This protein is thought to interact with the L protein, virus RNA and/or other N proteins.

The NSs protein refers to the protein of SEQ ID NO: 72 or a functional variant thereof. The protein of SEQ ID NO: 72 and its functional variants have the general properties of a bunyavirus NSs protein, taking as reference Bunyamwera (BUNV-NSs: GenBank NP_047214.1). The bunyavirus NSs protein plays a role in the escape of host innate immune response by promoting the degradation of host EIF2AK2/PKR and inhibiting host transcription. Cytoplasmic NSs interacts with host FBXW11 to degrade PKR whereas nuclear pool binds to host FBXO3 to target TFIIH subunit GTF2H1 for proteasomal degradation.

The second nucleic acid corresponds to the M segment which codes for the two virion glycoproteins, Gn (G2) and Gc (G1), and a nonstructural protein NSm, in the form of a polyprotein precursor (polyprotein M or M polyprotein) which is cotranslationally cleaved. The gene order is 5'Gn-NSm-Gc-3'. The polyprotein M coding sequence or ORF is from positions 1 to 4395 of SEQ ID NO: 3.

The polyprotein M refers to the protein precursor of SEQ ID NO: 4 which is cotranslationally cleaved into the two virion glycoproteins, Gn (G2) and Gc (G1), and nonstructural protein NSm, or a functional variant thereof. Gn is from positions 17 to 301 of SEQ ID NO: 4 and has the sequence SEQ ID NO: 58; NSm is from positions 302 to 475 of SEQ ID NO: 4 and has the sequence SEQ ID NO: 59, and Gc is from positions 476 to 1464 of SEQ ID NO: 4 and has the sequence SEQ ID NO: 60. The polyprotein of SEQ ID NO: 4, the derived Gn (G2), Gc (G1), and NSm proteins and their functional variants have the general properties of bunyavirus M polyprotein, Gn, Gc and NSm proteins, taking as reference Bunyamwera (BUNV-M: Uniprot P04505-1 or GenBank NP_047212.1). Using BUNV polyprotein M (1433 aa) as reference sequence, Gn is from positions 17 to 302, NSm is from positions 303 to 477, and Gc is from positions 478 to 1433. Glycoprotein N (Gn) and Glycoprotein C (Gc) interact with each other and are present at the surface of the virion. They are able to attach the virion to a cell receptor and to promote fusion of membranes after endocytosis of the virion. Non-structural protein M (NSm) plays a role in virion budding at Golgi tubes and in subcellular location of Glycoprotein C.

The third nucleic acid corresponds to the L segment which codes for the RNA-dependent or RNA-directed RNA polymerase (L protein) also named as large structural protein, Replicase, Transcriptase or RdRp. The L protein coding sequence or ORF is from positions 1 to 6828 of SEQ ID NO: 5.

The polymerase (L) protein refers to the protein of SEQ ID NO: 6 or a functional variant thereof. The protein of SEQ ID NO: 6 and its functional variants have the general properties of a bunyavirus L protein, taking as reference Bunyamwera (BUNV-L: UniProt P20470-1). The bunyavirus RNA-dependent RNA polymerase (RdRp or L protein) is responsible for replication and transcription of the viral RNA genome using antigenomic RNA as an intermediate. During transcription, it synthesizes subgenomic RNAs and assures their capping by a cap-snatching mechanism. These short capped RNAs are then used as primers for viral transcription. Bunyavirus RdRp have eight conserved motifs (PreA:F, A, H, B, C, D and E) in their central region or core polymerase domain (positions 951 to 1220 of BUN-L). The core polymerase domain of Moissiacense virus RdRP including the eight conserved motifs is from positions 954 to 1246 of SEQ ID NO: 6.

The percent amino acid or nucleotide sequence identity is defined as the percent of amino acid residues or nucleotides in a Compared Sequence that are identical to the Reference Sequence after aligning the sequences and introducing gaps if necessary, to achieve the maximum sequence identity. Alignment for purposes of determining percent amino acid or nucleotide sequence identity can be achieved in various ways known to a person of skill in the art, for instance using publicly available computer software such as the GCG (Genetics Computer Group, Program Manual for the GCG Package, Version 7, Madison, Wisconsin) pileup program, or any of sequence comparison algorithms such as BLAST (Altschul et al., J. Mol. Biol., 1990, 215, 403-10), FASTA or CLUSTALW.

Individual refers to a mammal, including a human or a domestic animal, preferably a human.

As used herein "the diagnosis of Moissiacense virus infection" includes the diagnosis of any Moissiacense virus associated disease, in particular encephalitis.

The terms "a", "an", and "the" include plural referents, unless the context clearly indicates otherwise. For example "a target" as used herein is understood to represent one or more targets. As such, the term "a" (or "an"), "one or more" or "at least one" can be used interchangeably herein.

In some embodiments, the S segment comprises a sequence having at least 85% or more identity with SEQ ID NO: 1 (said sequence may have at least 85%, 86%, 87%, 88%, 89%, 90%, 91%, 92%, 93%, 94%, 95%, 96%, 97%, 98% or 99% identity with SEQ ID NO: 1; or 100%° identity with SEQ ID NO: 1); the M segment comprises a sequence having at least 85% or more identity with SEQ ID NO: 3 (said sequence may have at least 85%, 86%, 87%, 88%, 89%, 90%, 91%, 92%, 93%, 94%, 95%, 96%, 97%, 98% or 99% identity with SEQ ID NO: 3; or 100%° identity with SEQ ID NO: 3); and/or the L segment comprises a sequence having at least 85% or more identity with SEQ ID NO: 5 (said sequence may have at least 85%, 86%, 87%, 88%, 89%, 90%, 91%, 92%, 93%, 94%, 95%, 96%, 97%, 98%, or 99% identity with SEQ ID NO: 5; or 100% identity with SEQ ID NO: 5).

In some embodiments, the nucleocapsid (N) protein has at least 90% identity with SEQ ID NO: 2 (said protein may have at least 90%, 91%, 92%, 93%, 94%, 95%, 96%, 97%, 98%, or 99% identity with SEQ ID NO: 2; or 100% identity with SEQ ID NO: 2 )°; the polyprotein M has at least 90% identity with SEQ ID NO: 4 (said protein may have at least 90%, 91%, 92%, 93%, 94%, 95%, 96%, 97%, 98%, or 99% identity with SEQ ID NO: 4; or 100% identity with SEQ ID NO: 4) and comprises the Gn protein from positions 17 to 301, the NSm from positions 302 to 475 and the Gc protein from positions 476 to 1464, the indicated positions being determined by alignment with SEQ ID NO: 4; and/or the L polyprotein has at least 90% identity with SEQ ID NO: 6 (said protein may have at least 90%, 91%, 92%, 93%, 94%, 95%, 96%, 97%, 98% or 99% identity with SEQ ID NO: 6; or 100% identity with SEQ ID NO: 6) including at least 90% identity on the core polymerase domain (positions 954 to 1246 of SEQ ID NO : 6). In some preferred embodiments, the nucleocapsid (N) protein has at least 95% identity with SEQ ID NO: 2 (said protein may have at least 95%, 96%, 97%, 98%, or 99% identity with SEQ ID NO: 2; or 100% identity with SEQ ID NO: 2)°; the polyprotein M has at least 90% identity with SEQ ID NO: 4 (said protein may have at least 90%, 91%, 92%, 93%, 94%, 95%, 96%, 97%, 98%, or 99% identity with SEQ ID NO: 4; or 100% identity with SEQ ID NO: 4) and comprises the Gn protein from positions 17 to 301, the NSm from positions 302 to 475 and the Gc protein from positions 476 to 1464, the indicated positions being determined by alignment with SEQ ID NO: 4; and the L polyprotein has at least 95% identity with SEQ ID NO: 6 (said protein may have at least 95%, 96%, 97%, 98% or 99% identity with SEQ ID NO: 6; or 100% identity with SEQ ID NO: 6) including at least 90% identity on the core polymerase domain (positions 954 to 1246 of SEQ ID NO : 6).

The method of the invention is used for the detection or diagnosis of any virus of the Moissiacense group or species, in particular Moissiacense virus, Marna virus, Umbre virus, Little Sussex virus or any variant thereof. In some preferred embodiments, said virus is Moissiacense virus, Marna virus or any variant thereof. Moissiacense virus has a S segment comprising SEQ ID NO: 1 which codes for a Nucleocapsid (N) protein of SEQ ID NO: 2 and a NSs protein of SEQ ID NO: 72, a M segment comprising SEQ ID NO: 3 which codes for a polyprotein M of SEQ ID NO: 4, and a L segment comprising SEQ ID NO: 5 which codes for a polymerase protein (L) of SEQ ID NO: 6. Marna virus has a S segment comprising SEQ ID NO: 90 which codes for a Nucleocapsid (N) protein of SEQ ID NO: 93 and a NSs protein of SEQ ID NO: 94, a M segment comprising SEQ ID NO: 91 which codes for a polyprotein M of SEQ ID NO: 95, and a L segment comprising SEQ ID NO: 92 which codes for a polymerase protein (L) of SEQ ID NO: 96. Umbre virus includes the strain IG1424 comprising a S segment having the sequence GenBank accession number KP792685.2 as accessed on November 16, 2016 which codes for a Nucleocapsid (N) protein having the sequence GenBank: AKO90197.1 as accessed on November 16, 2016 and a NSs protein having the sequence GenBank: AKO90198.1 as accessed on November 16, 2016 ; a M segment having the sequence GenBank accession number KP792686.1 as accessed on December 01, 2015 which codes for a polyprotein M having the sequence GenBank: AKO90182.1 as accessed on December 01, 2015 ; and a L segment having the sequence GenBank accession number KP792687.1 as accessed on December 01, 2015 which codes for a polymerase protein (L) having the sequence GenBank: AKO90167.1 as accessed on December 01, 2015. Little Sussex virus has a L segment comprising the sequence GenBank accession number KT720483.1 as accessed on April 02, 2016 which codes for a polymerase protein (L) comprising having the sequence GenBank: AMR73388.1 as accessed on April 02, 2016.

The viral nucleic acid or target nucleic acid may be genomic RNA (viral RNA or vRNA), complementary RNA (cRNA) or viral mRNA. Genomic RNA or viral RNA (vRNA) is negative-sense RNA comprising the reverse complement of one of the above-mentioned coding sequences, in the RNA form. Complementary RNA (cRNA) is positive-sense RNA comprising one of the above-mentioned coding sequences, in the RNA form. Viral mRNA comprises one of the above-mentioned coding sequences, in the RNA form. In some preferred embodiments of the methods of the invention, the viral nucleic acid is viral RNA (*i.e*. Moissiacense virus genomic RNA).

The diagnosis or detection method of the invention comprises the detection of a target sequence of the viral nucleic acid. The target sequence refers to the particular nucleotide sequence of the viral nucleic acid that is to be detected by any appropriate mean such as hybridization with an oligonucleotide probe, amplification with a pair of oligonucleotide primers, sequencing, in particular high-throughput sequencing, or a combination thereof. For example, an amplification product (amplicon) of the target sequence obtained by amplification of viral nucleic acid from the sample may be detected by using a DNA binding dye or labelled primer, or hybridization assay using a labelled oligonucleotide probe. As used herein, the detection of a target sequence of the viral nucleic acid refers to the direct detection and the detection following another step performed on the viral nucleic acid such as a reverse transcription step and an amplification of said viral nucleic acid.

The target sequence includes the sequence to which oligonucleotide probe hybridizes during the nucleic acid detection process and the sequences to which oligonucleotide primers hybridize during the nucleic acid amplification process. The target sequence that is detected directly using an oligonucleotide probe consists usually from a lower limit of 15 to 20 consecutive nucleotides to an upper limit of 45 to 60 consecutive nucleotides of one of the above-mentioned viral nucleic acid sequences. The target sequence that is amplified and detected consists usually of at least 50 consecutive nucleotides, preferably at least 100 or 150 consecutive nucleotides of one of the above-mentioned viral nucleic acid sequences. Preferably, the target sequence consists of 150 to 600 consecutive nucleotides, more preferably 300 to 500 consecutive nucleotides of one of the above-mentioned viral nucleic acid sequences. The target nucleic acid being originally single-stranded, the term target sequence will also refer to the sequence complementary to the target sequence as present in the viral nucleic acid.

The target sequence is a unique sequence which is specific for Moissiacense virus. Thus, the detection of the target sequence indicates the presence of Moissiacense virus in the sample. Therefore, the detection of the target sequence in a sample from the individual is indicative of whether the individual is suffering from Moissiacense virus infection and in particular from a disease caused by Moissiacense virus such as encephalitis.

By comparing the L, M and S segment sequences of Moissiacense virus which are disclosed in the present application with that of other bunyavirus species or other members of the order *Bunyavirales* which are available in the sequence databases, one skilled in the art can easily identify a unique target sequence which is specific for Moissiacense virus using standard nucleotide sequence analysis softwares that are well-known in the art.

In some embodiments, the method of the invention comprises the detection of at least one target sequence selected from the group consisting of:
a) a sequence SEQ ID NO: 7 situated in the region from positions 23 to position 338 of the S segment sequence SEQ ID NO: 1;
b) a sequence SEQ ID NO: 8 situated in the region from positions 3706 to 4130 of the M segment sequence SEQ ID NO: 3;
c) a sequence SEQ ID NO: 9 situated in the region from positions 3312 to 3768 of the L segment sequence SEQ ID NO: 5;
d) a sequence of at least 15 nucleotides, preferably at least 50, 100, 150, 200 or 300 nucleotides from any one of SEQ ID NO: 7 to 9; and
e) a sequence comprising any one of SEQ ID NO: 7 to 9 and up to 40 consecutive nucleotides, preferably up to 35, 30, 25 or 20 (2, 3, 4, 5, 6, 7, 8, 9, 10, 11, 12, 13, 14 ,15, 16, 17, 18, 19 and 20), more preferably up to 15 (2, 3, 4, 5, 6, 7, 8, 9, 10, 11, 12, 13, 14 ,15), up to 10 (2, 3, 4, 5, 6, 7, 8, 9, 10) or up to 5 (2, 3, 4, 5) consecutive nucleotides of 5' and/or 3' flanking sequence from said S, M or L segment sequence.

The target sequences listed above are species specific. They allow the detection of viruses of the Moissiacense group or species including at least Moissiacence, Marna, Umbre and Little Sussex viruses. The target sequence consists of or is included, comprised in or contained in the target region. In some preferred embodiment, the target sequence consists of the target region as defined above.

Nucleic acid amplification techniques include target amplification systems, probe amplification systems and signal amplification. Target amplification systems include with no limitations: Polymerase Chain Reaction (PCR) including RT-PCR, nested PCR and multiplex PCR; Nucleic Acid Sequence-Based Amplification (NASBA), Transcription Mediated Amplification (TMA), Strand Displacement Amplification (SDA) and isothermal amplification such as reverse-transcription loop-mediated isothermal amplification (RT-LAMP). Target nucleic acid amplification is performed using any of the various natural or engineered enzymes available for this purpose. For example, PCR can be performed using any thermostable polymerase such as with no limitations: Taq, Vent, Pfu, Tth and variants thereof. Signal amplification includes Branched DNA probes (bDNA) and Hybrid capture assay using anti-DNA-RNA hybrid antibody conjugated to a label such as phosphatase alkaline. Probe amplification includes Ligase Chain Reaction (LCR) and Cleavase Invader (FEN-1 DNA polymerase (cleavase)). Hybridization is preferably sequence-specific hybridization, i.e., hybridization with a nucleic acid probe that specifically hybridizes to a target sequence specific for Moissiacense virus.

Since the viral nucleic acid is RNA, the method of the invention comprise a reverse transcription reaction prior to or concomitantly with the amplification reaction of the target sequence. The reverse transcription reaction is performed using any reverse transcriptase (RT). RT are well-known in the art and include for example Avian Myeloblastosis Virus (AMV) and Moloney Murine Leukemia virus (MMLV) RT. The Reverse transcription and DNA amplification can be performed in the same reaction mixture comprising the DNA polymerase and RT enzymes.

The amplification product is detected using any of the various methods available for this purpose which are well-known in the art. For example, the detection method is fluorescence, bioluminescence, colorimetry or immunoenzymatic detection. The detection may be semi-quantitative or quantitative. The detection may also be real-time detection, wherein the signal resulting from the presence of the amplification product is measured during the course of the nucleic acid amplification reaction to monitor the accumulation of specific amplification products. Fluorescence may use DNA intercaling dyes or fluorescent molecular beacon probes. Immunoassays include Enzyme-linked immunosorbent assays (ELISA) and lateral flow.

In some preferred embodiments, the method of the invention comprises:
- subjecting said sample to a nucleic acid amplification reaction using at least one pair of oligonucleotide primers for amplifying a target sequence of said viral nucleic acid, and
- detecting the presence of an amplification product for said target sequence.

The target amplification reaction is performed using any nucleic acid target amplification techniques as defined above. In some preferred embodiments, target amplification is performed by RT-PCR techniques using suitable enzymes for that purpose as defined above. The amplification product includes monomers and concatemers of the target sequence. The amplification product may be detected using any appropriate means available for that purpose as disclosed above such as by using a labelled primer or probe or a DNA binding dye. In the second step of the method of the invention, the detection of the presence or absence of the amplification product determines the presence or absence of a Moissiacence virus in the sample, and thereby whether or not the individual is infected with Moissiacense virus.

Oligonucleotide refers to a polymer of 5 to 100 nucleotides, preferably from a lower limit of 15 to 20 nucleotides to an upper limit of 45 to 60 nucleotides, wherein the polymer comprise ribonucleotides, deoxyribonucleotides, modified nucleotides or mixtures thereof and may further include modified internucleotide linkages and/or modified 5' and/or 3' termini. For example, the oligonucleotide can be DNA, RNA, PNA or mixed, and may comprise locked nucleic acids (LNA). Oligonucleotides are usually synthesized using any of a variety of well-known enzymatic or chemical methods. Oligonucleotide includes a probe or primer. The oligonucleotide probe or primer according to the invention is substantially complementary to the target sequence. Substantially complementary means that the oligonucleotide is at least 80% identical, preferably at least 85%, 90%, 95%, 96%, 97% 98% or 99% identical to the target sequence. The oligonucleotide may comprise additional sequences (not complementary to the target sequence) at its 5' end. In some embodiments the oligonucleotide comprises a sequence of at least 5, preferably 10 to 15 consecutive nucleotides which is 100% identical to the target sequence. In some more preferred embodiments, the oligonucleotide sequence is 100% identical to the target sequence.

The oligonucleotides which function as probes are capable of hybridizing specifically to the target sequence. They advantageously include a label to detect the target nucleic acid. The oligonucleotides which function as primers are capable of annealing specifically to the target sequence and can be further extended in the presence of a nucleic acid polymerase to specifically amplify the target sequence. At least one oligonucleotide may also function as a probe and further includes a detectable label to detect a target nucleic acid or an amplicon thereof.

The label is any moiety that can be detected directly or indirectly by the production of a detectable signal such as luminescent (radioluminescent, chemiluminescent, bioluminescent, fluorescent or phosphorescent), radioactive, colorimetric, magnetic signal or others. Directly detectable labels include radioisotopes and fluorophores. Indirectly detectable labels are detected by labelling with additional reagents that enable the detection. Indirectly detectable labels include, for example, chemiluminescent agents, enzymes that produce visible or coloured reaction products. Indirectly detectable labels also include ligand-detectable ligand binding partners, for example based on avidin or streptavidin/biotin complex, such as with no limitations a biotinylated oligonucleotide (for nucleic acid detection) or antibody (for protein detection) and streptavidin conjugated with a label (fluorophore or enzyme).

The oligonucleotides are designed by comparing the L, M and S segment sequences of the Moissiacense virus which are disclosed in the present application with that of other bunyaviruses or other members of the order *Bunyavirales* which are available in sequence databases, using general principles for designing amplification primers or probes that are well-known in the art. The design of the primers may also include specific principles for designing primers for use in specific amplification methods. Appropriate softwares for designing oligonucleotide primers or probes are available in the art. The sequences of the oligonucleotide primers or probe are specific for the target sequence of the Moissiacense virus. This means that they are chosen to hybridize specifically to the target sequence of the Moissiacense virus but not to related target sequences of other bunyaviruses or other members of the order *Bunyavirales* which infect humans or animals, under the hybridization conditions which are used for the methods of the invention. It is within the skills of one of ordinary skill in the art to choose the sequences of the oligonucleotide probe or primes pair and adapt the hybridization conditions (Temperature, salt concentration) of said oligonucleotide probe or primer pair to permit only hybridization with nucleic acid of Moissiacense virus.

In some embodiments of the above method, the oligonucleotide probe or primers are selected from the group consisting of: the sequences of 15 to 100 nucleotides, preferably 15 to 60 nucleotides having 80% to 100% identity, preferably at least 85%, 90% 95%, 96 %, 97%, 98% or 99% identity with any of SEQ ID NO: 1, 3, 5, and 7 to 9 or with a sequence comprising any of SEQ ID NO: 7 to 9 and up to 40 consecutive nucleotides, preferably up to 35, 30, 25 or 20 (2, 3, 4, 5, 6, 7, 8, 9, 10, 11, 12, 13, 14 ,15, 16, 17, 18, 19 and 20), more preferably up to 15 (2, 3, 4, 5, 6, 7, 8, 9, 10, 11, 12, 13, 14 ,15), up to 10 (2, 3, 4, 5, 6, 7, 8, 9, 10) or up to 5 (2, 3, 4, 5) consecutive nucleotides of 5' and/or 3' flanking sequence from said S, M or L segment sequence, and their complement.

In some preferred embodiments, the oligonucleotide probe or primers are selected from the group consisting of the sequences SEQ ID NO: 10 to 57. The primer pairs are advantageously selected from the group consisting of: SEQ ID NO: 10-11, 12-13, 14-15, 16-17, 18-19, 20-21, 22-23, 24-25, 26-27, 28-29, 30-31, 32-33, 34-35, 36-37, 38-39, 40-41, 42-43, 44-45, 46-47, 48-49, 50-51, 52-53, 54-55, and 56-57. The primer pairs SEQ ID NO: 10-11, 12-13, and/or 14-15 are advantageously used for the detection of any virus of the Moissiacense group, in particular Moissiacense virus, Umbre virus, Little Sussex virus or any variant thereof.

### Antigen or antibody detection

In some aspects, the method of detection or diagnosis of Moissiacense virus comprises determining the presence of at least one protein of said virus or antibodies thereto in a sample.

The detection or diagnosis is generally performed by immunoassay using an antigen from virus Moissiacense or antibody thereto. Immunoassays are well-known techniques for protein or antibody detection which rely on the detection of antigen-antibody complexes using an appropriate label. The method of the invention may use any immunoassay such as with no limitations, immunoblotting, immunoprecipitation, ELISA, immunocytochemistry or immunohistochemistry, and immunofluorescence like flow cytometry assay, and FACS. The method of the invention may use any appropriate label used in immunoassays such as enzymes, biotin, fluorescent dyes/proteins or others.

In some embodiments, the antigen from Moissiacense virus comprises the amino acid sequence of the N, Gn or Gc protein or a fragment thereof. A fragment refers to a functional fragment, which means a fragment of at least 10 amino acids that is bound by antibodies against Moissiacense virus. In some embodiments, the fragment comprises at least 50, 100, 200, 300 or more amino acids.

In some preferred embodiments, the antigen comprises the N protein amino acid sequence or a fragment thereof, preferably a sequence having 90% to 100% identity with SEQ ID NO: 2, or a fragment of at least 10 amino acids thereof which is bound by antibodies against Moissiacense virus.

In some other preferred embodiments, the antigen comprises an extracellular fragment of Gc protein, preferably chosen from:
- a sequence SEQ ID NO: 61 comprising the extracellular domain (positions 476 to 1395 on the polyprotein M of SEQ ID NO: 4);
- a sequence SEQ ID NO: 62 comprising the N-terminal alpha-helix (positions 510 to 770 on the polyprotein M of SEQ ID NO: 4);
- a sequence SEQ ID NO: 63 comprising the N-terminal domain (positions 477 to 907 on the polyprotein M of SEQ ID NO: 4), and
- a sequence of at least 50 amino acids from any one of SEQ ID NO: 61 to 63, in particular SEQ ID NO: 64 (positions 477 to 730 of SEQ ID NO: 4).

In some embodiments, the method is for the detection or diagnosis of Moissiacense virus, Marna virus or any variant thereof.

An antibody refers to an antibody against a protein of Moissiacense virus, preferably the N, Gn or Gc protein, more preferably an antibody which binds an extracellular epitope of the Gn or Gc protein that is exposed on the surface of virion particles. The antibody according to the invention recognizes specifically said viral protein, in particular Gn or Gc protein on the plasma membrane of infected cells. Such recognition can be determined by standard immunofluorescence assay like flow cytometry assay. The antibody has a relatively high affinity to its epitope of said viral protein, but do not substantially recognize and bind to peptides other than the one(s) of interest. As used herein, the term "relatively high affinity" means a binding affinity between the antibody and the protein of interest of at least 10⁻⁶ M, and preferably of at least about 10⁻⁷ M and even more preferably 10⁻⁸ M to 10⁻¹⁰ M. Determination of such affinity is preferably conducted under standard competitive binding immunoassay conditions which is common knowledge to the person of ordinary skill in the art. The term "antibody" is meant to encompass a whole antibody or antigen binding fragment thereof, an aggregate, polymer, derivative, or conjugate of antibody. The antibody may be monoclonal, polyclonal, non-recombinant or recombinant, chimeric or humanized.

The antigen or antibody may include an appropriate label for antibody or antigen detection, for example in the form of a fusion protein or conjugate. In some embodiments, the antigen is fused with an enzyme such as luciferase.

The antigen of the invention is prepared by the conventional techniques known to those skilled in the art, in particular by expression from an appropriate recombinant expression vector in a suitable cell system (eukaryotic including mammalian and insect cells or prokaryotic). In particular embodiments, the vector comprises any one of SEQ ID NO: 65 to 68 and 73 or a fragment thereof

The antibody of the invention can be produced by the conventional techniques known to those skilled in the art. For example, they may be produced by immunization of an animal with an antigen from virus Moissiacense, in particular a recombinant antigen as define above.

The antigen and antibody are specific for Moissiacense virus and usually do not exhibit substantial cross-reactivity with other pathogens that infect human or animal individuals, under the conditions for antigen-antibody complex formation that are used in the methods of the invention. It is within the skill of a person having ordinary skill in the art to prepare specific antigen and antibodies and adapt the conditions of the method to detect the specific binding of the diagnostic agent (antigen or antibody) to its antibody or antigen counterpart from Moissiacense virus.

In some embodiments, the method of detection or diagnosis of Moissiacense virus comprises the step of:
- incubating an antigen from Moissiacense virus or antibody thereto with the biological sample to form a mixture; and
- detecting antigen-antibody complexes in the mixture.

The sample for anti-Moissiacense virus antibody detection is preferably body fluid from the individual, in particular serum. The sample for viral protein detection may be tissue sample, in particular biopsied tissue such as brain biopsy, or body fluid such as cerebral spinal fluid (CSF), whole-blood or serum.

The antigen or antibody is preferably labeled and the antigen-antibody complexes are detected by measuring the signal from the label by any appropriate means available for that purpose as disclosed above.

In some embodiments, antibody detection is performed by luciferase immunoprecipitation using an antigen fused with luciferase, preferably an antigen comprising SEQ ID NO: 61 or 62.

In some embodiments, antibody detection is performed by ELISA using an antigen comprising SEQ ID NO: 63 or 64.

In some embodiments, the detecting step comprises the determination of the amount of bound antibody or bound antigen in the mixture, and optionally, comparing the amount of bound antibody or bound antigen in the mixture with at least one predetermined value.

The detection of the viral nucleic acid target sequence, protein or antibody thereto in a sample from the individual using the methods of the invention is indicative of whether the individual is suffering from Moissiacense virus infection and in particular from a disease caused by Moissiacense virus such as encephalitis.

Therefore, the above methods of the invention are useful for the diagnosis of Moissiacense virus infection in an individual, in particular the diagnosis of the disease caused by Moissiacense virus, ranging from febrile illness to central nervous system involvement such as encephalitis. In some embodiments, said virus is Moissiacense virus, Marna virus or any variant thereof.

In some embodiments, the above methods comprise the step of deducing therefrom whether the individual is suffering from Moissiacense virus infection and in particular from a disease caused by Moissiacense virus such as encephalitis.

In some embodiments in connection with this aspect of the invention, the above methods comprise a further step of administering an appropriate treatment to the individual depending on whether or not the individual is diagnosed with Moissiacense virus infection and in particular with a disease caused by Moissiacense virus such as encephalitis.

The above methods of the invention are in particular useful for the differential diagnosis of encephalitis, in particular human encephalitis. In some embodiments, in connection with this aspect of the invention, the same biological sample from an individual may be subjected, simultaneously or separately, to a method of detection or diagnosis of at least another pathogen, in particular at least another encephalitis-causing agent such as encephalitis virus. Encephalitis virus includes in particular Herpesvirus such as HSV and VZV, enteroviruses, polyomaviruses, astroviruses, measles virus, mumps virus, and various arboviruses such as without limitation, West Nile, JEV and TBEV.

The above methods of the invention are also useful for the detection of Moissiacense virus in an arthropod vector population to study virus Moissiacense dissemination across geographic areas for epidemiological surveys.

In some preferred embodiments, the above methods of the invention comprise assaying several samples simultaneously, and/or assaying different target sequences, antigens or antibodies simultaneously on the same sample, in parallel in separate reaction mixtures, or in the same reaction mixture and detected independently. In some advantageous embodiments, the methods comprise assaying multiple samples, targets, antigens, and/or antibodies simultaneously in a high-throughput process.

### Kits for the diagnosis or detection of Moissiacense virus

Another aspect of the invention is a kit for the diagnosis or detection of Moissiacense virus, comprising at least one oligonucleotide probe or primer, antigen or antibody thereto for the detection of Moissiacense virus nucleic acid, antibody or protein, as defined above, preferably further including a detectable label. In particular, the kit comprises at least one pair of oligonucleotide primers for amplifying said target; preferably wherein at least one oligonucleotide of the pair may comprise a detectable label. In some embodiments, the kit is for the diagnosis or detection of Moissiacense virus, Marna virus or any variant thereof.

In some embodiments, the kit is for the differential diagnosis of encephalitis, in particular human encephalitis. In some particular embodiments, in connection with this aspect of the invention, the kit further comprises at least one oligonucleotide probe or primer, antigen or antibody thereto for the detection of least another encephalitis-causing agent such as encephalitis virus, as defined above. The encephalitis virus is in particular chosen from Herpesvirus such as HSV and VZV, enteroviruses, polyomaviruses, astroviruses, measles virus, mumps virus, and various arboviruses such as without limitation, West Nile, JEV and TBEV. The at least one oligonucleotide probe or primer, antigen or antibody for the detection of said other(s) encephalitis-causing agent(s) preferably further includes a detectable label. In particular, the kit comprises at least one pair of oligonucleotide primers for amplifying a target sequence of the nucleic acid of at least another encephalitis-causing agent such as encephalitis virus as defined above; preferably wherein at least one oligonucleotide of the pair may comprise a detectable label.

The kit optionally comprises reagents for the amplification of the target sequence and/or the detection of the amplification product or antigen/antibody complex. Reagents available for this purpose are well-known in the art and include the DNA polymerases and RT enzymes described above, buffers or substrates for the enzymes, detergents, enhancing agents, nucleic acid binding dyes and probes, preferably labelled probes, secondary antibody conjugated to a label, avidin/streptavidin conjugated to a label. In some preferred embodiments of the kit of the invention, the primers, probe, antigen or antibody, and optional reagents are in lyophilised form to allow ambient storage. The components of the kits are packaged together into any of the various containers suitable for nucleic acid amplification or antigen/antibody complex detection such as plates, slides, wells, dishes, beads, particles, cups, strands, chips, strips and others. The kit optionally includes instructions for performing at least one specific embodiment of the method of the invention. In some advantageous embodiments, the kit comprises micro-well plates or microtubes, preferably in a dried format, i.e., wherein the wells of the plates or microtubes comprise a dried composition containing at least the primers, probe, antigen or antibody, and preferably further comprising all the reagents for the amplification of the target sequence and/or the detection of the amplification product amplification or antigen/antibody complex. In some other advantageous embodiments, the primers, probe, antigen or antibody and optional reagents are included into any of the devices available for nucleic acid amplification or immunoassay including devices further integrating nucleic acid extraction and/or amplification product detection capacities such as microfluidic devices or other devices.

Another aspect of the invention, relates to an oligonucleotide probe or primer, antigen or antibody thereto for the detection of Moissiacense virus nucleic acid, antibody or protein, as defined above. The invention also relates to the use of an oligonucleotide probe or primer, antigen or antibody thereto for the detection of Moissiacense virus nucleic acid, antibody or protein as defined above, as diagnostic agent for the *in vitro* detection or diagnosis of Moissiacense virus. The invention encompasses combinations of the above diagnostic agents for the detection of Moissiacense virus with other diagnostic agents, in particular oligonucleotide probe(s) or primer(s), antigen(s) or antibodies thereto, for the detection of other encephalitis-causing agent(s) as defined above and their use for the differential diagnosis of encephalitis, in particular human encephalitis. In some embodiments, said virus is Moissiacense virus, Marna virus or any variant thereof.

### Immunogenic or vaccine compositions

Another aspect of the invention, relates to an immunogenic or vaccine pharmaceutical composition comprising, as active substance an antigen from Moissiacense virus as defined above, in association with at least one pharmaceutically acceptable vehicle.

The pharmaceutical vehicles are those appropriate to the planned route of administration, which are well known in the art.

The pharmaceutical composition may further comprise a carrier and/or adjuvant. Non-limitative examples of carriers suitable for use in the composition of the invention include uni- or multi-lamellar liposomes, ISCOMS, virosomes, viral pseudo-particules, saponin micelles, saccharid (poly(lactide-co-glycolide)) or gold microspheres, and nanoparticules. Non-limitative examples of adjuvants suitable for use in the composition of the invention include: CpG oligodeoxynucleotide, Freund adjuvant, polyLC (polyinosine-polycytidylic acid), oil emulsion, mineral substances, bacterial extracts, saponin, aluminium salts, monophosphoryl-lipid A and squalene.

The pharmaceutical composition comprises a therapeutically effective amount of the antigen sufficient to induce a protective immune response against Moissiacense virus infection in the individual to whom it is administered. The pharmaceutically effective dose depends upon the composition used, the route of administration, the type of mammal (human or animal) being treated, the physical characteristics of the specific mammal under consideration, concurrent medication, and other factors, that those skilled in the medical arts will recognize.

The invention provides also an antigen according to the invention for use as a medicament.

The invention provides also an antigen or pharmaceutical composition according to the invention for use in the prevention of Moissiacense virus infection and associated disease, in particular encephalitis. In some embodiments, said virus is Moissiacense virus, Marna virus or any variant thereof.

The invention provides also a method for preventing treating Moissiacense virus infection and associated disease, in particular encephalitis in an individual, comprising: administering a therapeutically effective amount of the pharmaceutical composition according to the invention to the individual. In some embodiments, said virus is Moissiacense virus, Marna virus or any variant thereof.

The pharmaceutical composition of the present invention is generally administered according to known procedures, at dosages and for periods of time effective to induce a beneficial effect in the individual. The administration may be by injection.

The practice of the present invention will employ, unless otherwise indicated, conventional techniques which are within the skill of the art. Such techniques are explained fully in the literature.

The invention will now be exemplified with the following examples, which are not limitative, with reference to the attached drawings in which:

### FIGURE LEGENDS

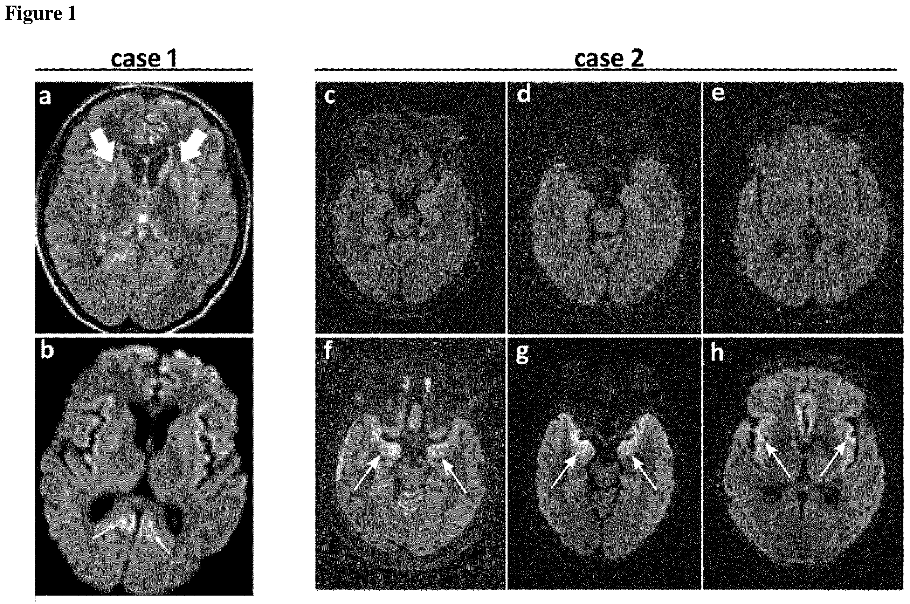

**Figure 1****: Brain MRI of the two patients with Umbre virus encephalitis**
   **a & b:** case 1. Axial FLAIR (a) and Diffusion-weighted **(b)** images, revealing mild hyper intensity of both putamina (thick arrows), and of frontal, insular and posterior cingulate cortices (thin arrows), **c to h:** case 2. Brain MRI at month 1 **(c to e)** and month 5 **(f to h); c & f:** axial T2; **d & e; g & h:** diffusion-weighted images, **c, d, e:** normal hippocampi and insular cortices at month **1. f, g, h:** bilateral hypersignal (arrows) of the hippocampi and insular cortices, with mild bi-hippocampi atrophy.

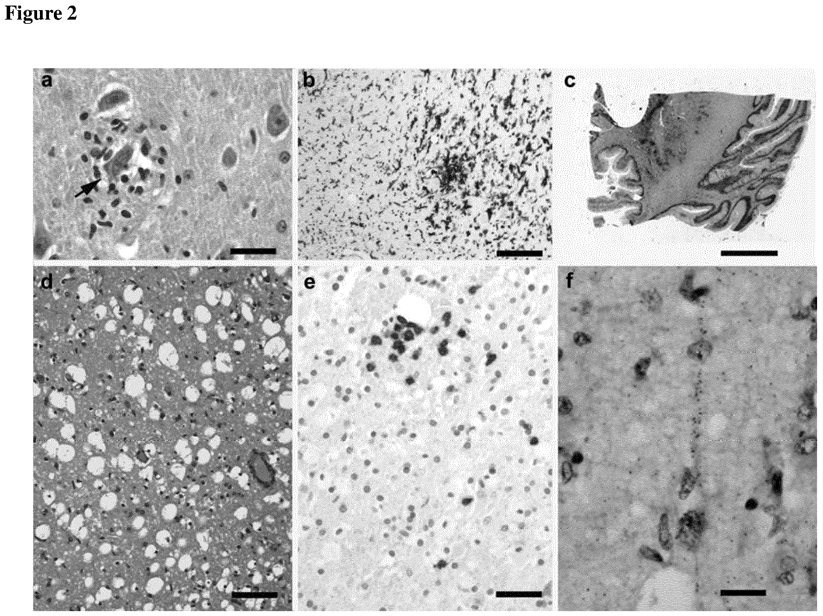

**Figure 2****: Histopathology in case 1**
   **a:** Parahippocampal cortex. Haematein-eosin. Microglial cells surrounding a neuron (black arrow), suggesting neuronophagia. **b:** Dentate nucleus. IBA-1 immunohistochemistry. Diffuse microglial activation and microglial nodule. **c:** Cerebellum. CD163 immunohistochemistry. Distribution of activated microglia. Enhancement in the dentate nucleus. **d:** Temporal cortex. Haematein-eosin. Spongiosis. **e:** Caudate nucleus. CD3 immunohistochemistry. Clusters of T-lymphocytes. **f:** Frontal cortex. In situ hybridization with a bunyavirus probe. Labeling (red) of the cell body and dendritic shaft of a pyramidal neuron. Scale bars: a & e = 50 µm; b & d = 100 µm; c = 5 mm; f = 25 µm.

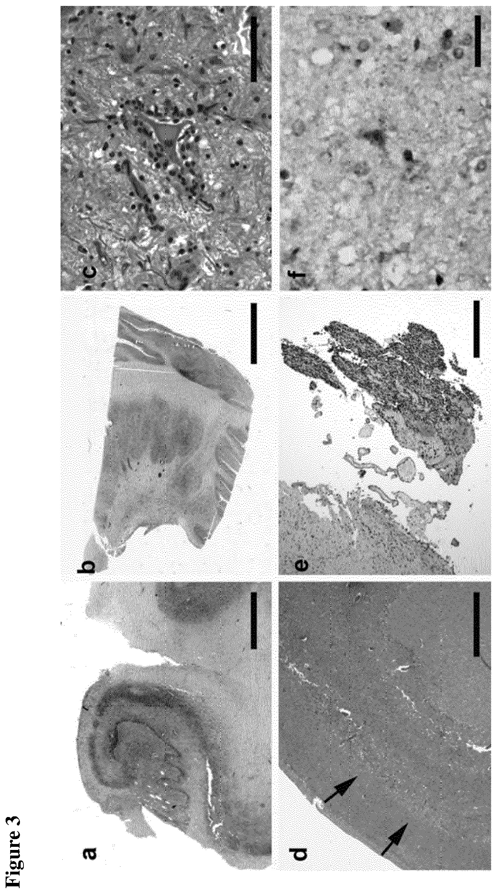

**Figure 3****: Histopathology in case 2**
   **a:** Right hippocampus. CD163 immunohistochemistry. Distribution of activated microglia. Enhancement of the dentate gyrus and pyramidal sectors of the cornu Ammonis. **b:** Cerebellum. CD163 immunohistochemistry. Distribution of activated microglia. Enhancement in the dentate nucleus and cerebellar cortex. c: Anterior horn of the spinal cord. Haematein-eosin. Microglial cells surrounding a neuron, suggesting neuronophagia. **d:** Right hippocampus. Haematein-eosin. Neuronal loss in CA1 (black arrows), **e:** Roots of the oculomotor nerve. CD163 immunohistochemistry. Infiltration by macrophages. **f:** Frontal cortex. In situ hybridization with a bunyavirus probe. Labeling (red) of the cell body of a pyramidal neuron.Scale bars: a & b = 5 mm; c = 50 µm; d = 100 µm; e = 500 µm; f = 25 µm.

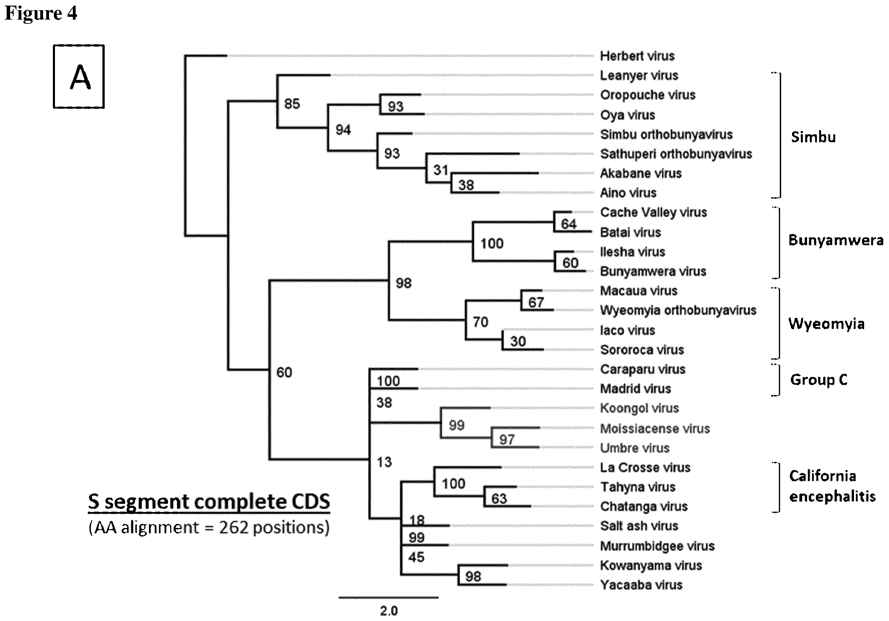

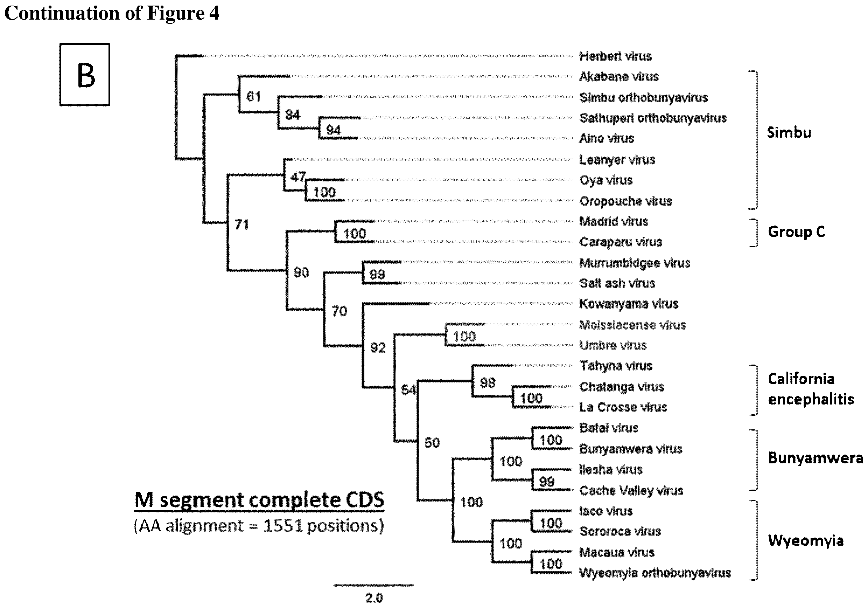

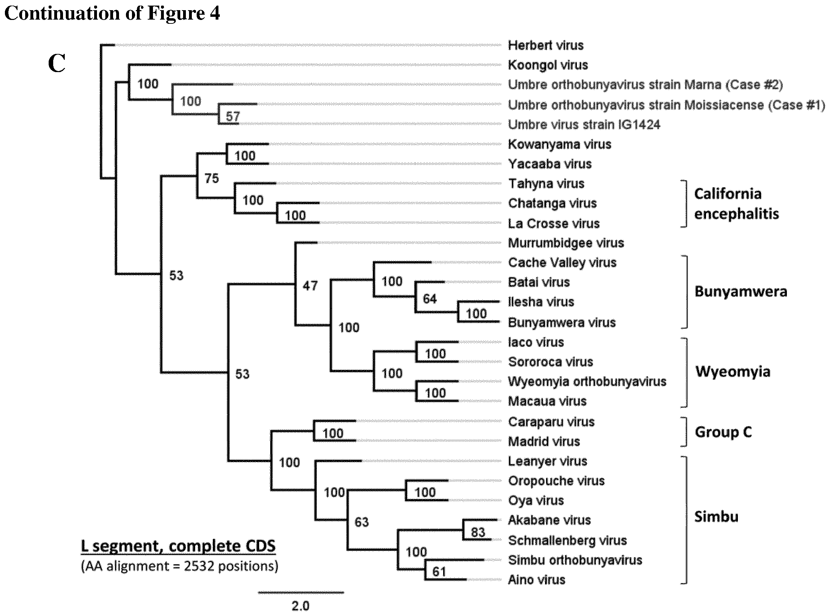

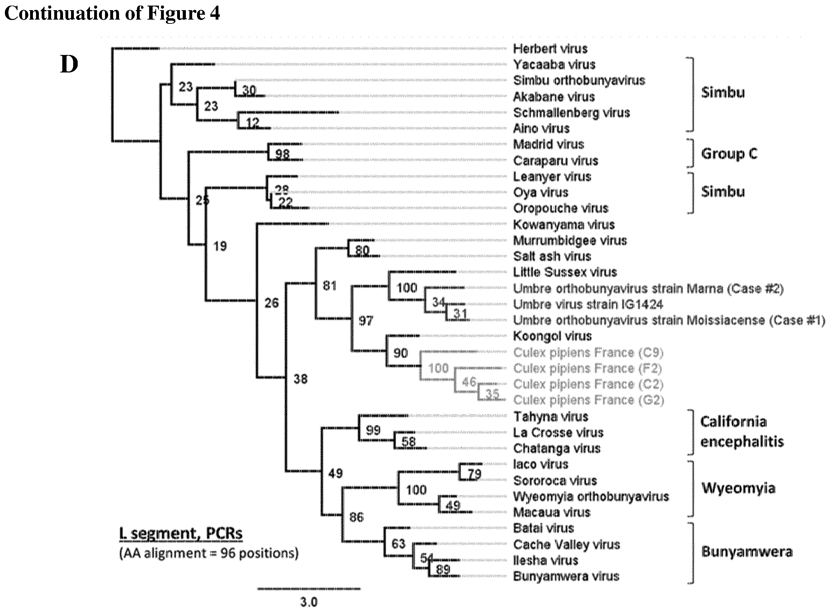

**Figure 4****: Phylogenetic analysis of orthobunyaviruses.**
   **A)** S-segment, **B)** M-segment, **C)** L-segment and **D)** PCR-targeted region on the L-segment. The bootstrap values are given at each branches. Moissiacense virus and the closest relative viral sequences are indicated in red. Serogroups are indicated in black on the right side of each panel. The details of the methods used for the phylogenetic analysis and the list of accession numbers are available in the material and methods section.

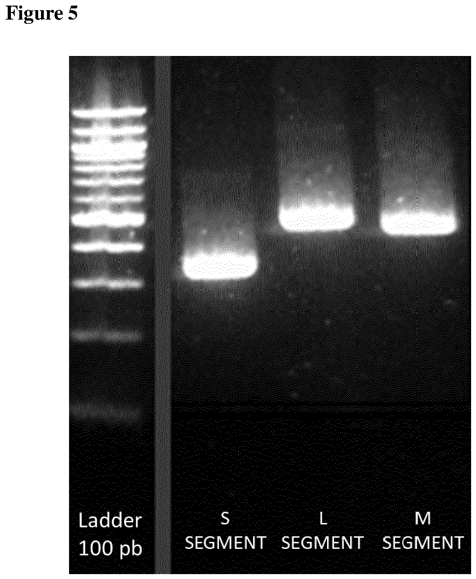

**Figure 5****:** RT-PCR assay on the patient's brain sample using primer pairs specific for Moissiacense virus species S, L and M segment sequences.

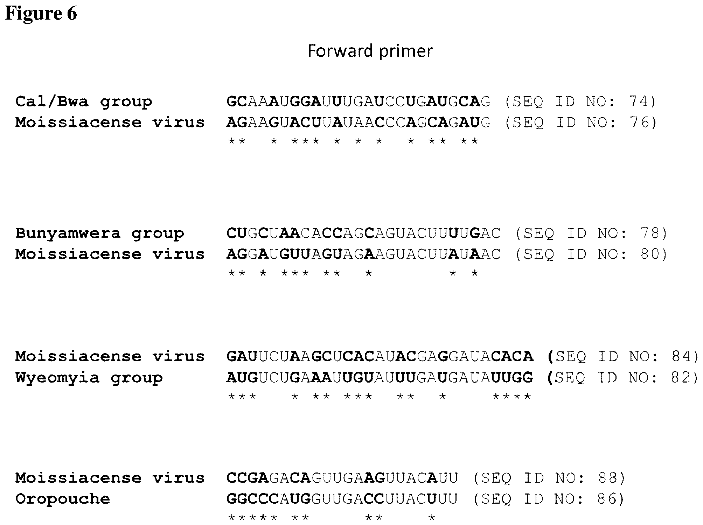

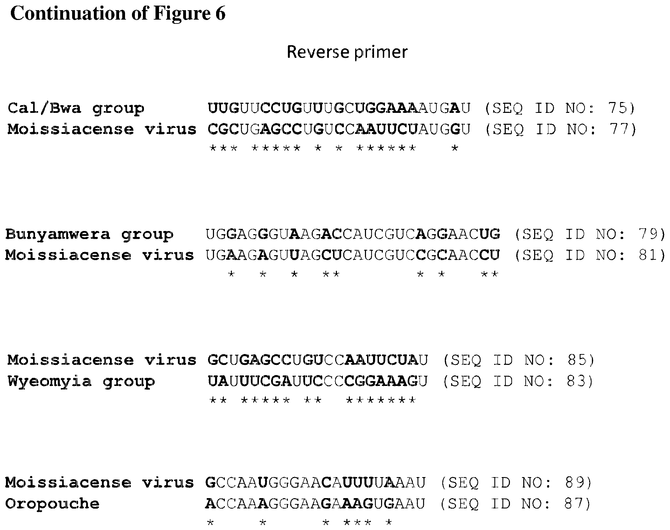

**Figure 6****:** Comparison of multiplex pan-orthobunyaviruses PCR primers from Lambert AJ & Lanciotti RS (J. Clin. Microbiol., 2009, 47, 2398-404) with Moissiacense virus sequence (SEQ ID NO: 74 to 89). Disagreements between the consensus primers and Moissiacense virus sequences are highlighted.

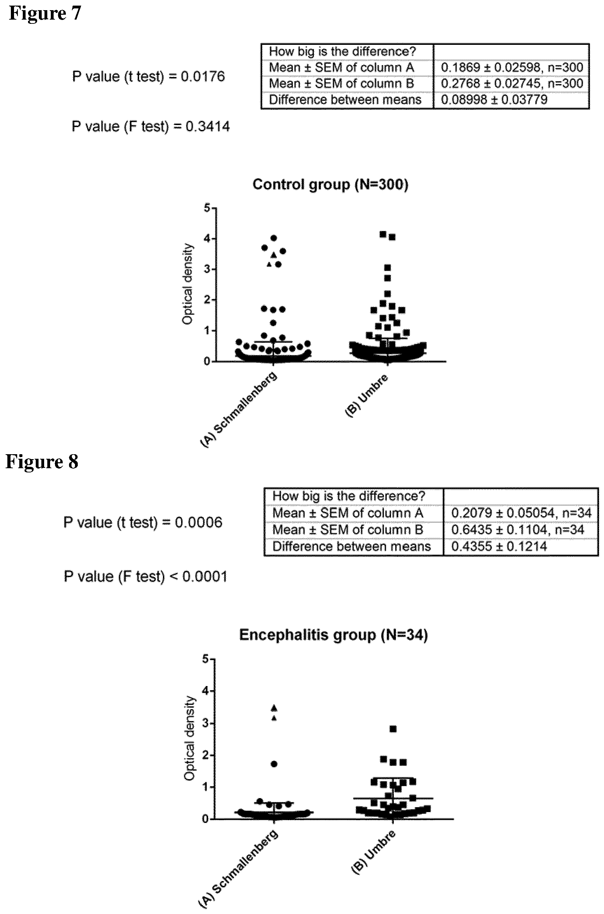

**Figure 7****:** Distribution of raw optical density values (ELISA immunoassay) using the Gc Head domain of Schmallenberg virus and Umbre virus as antigen on a control population (N=300). Black triangle: Schmallenberg positive serum tested undiluted. Small black triangle: Schmallenberg positive serum tested at a 10-fold dilution.
**Figure 8****:** Distribution of raw optical density values (ELISA immunoassay) using the Gc Head domain of Schmallenberg virus and Umbre virus as antigen on a encephalitis group (N=34). Black triangle: Schmallenberg positive serum tested undiluted. Small black triangle: Schmallenberg positive serum tested at a 10-fold dilution.

### EXAMPLES

### 1. Material and Methods

### - Metatranscriptomics analysis

By using untargeted metatranscriptomics, the inventors aimed at identify RNA sequences from bacteria, fungi, viruses or protozoans without prior hypothesis on the involved pathogen. RNA, rather than DNA, is indicative of living microorganisms. For each patient individually, total RNA was extracted from post-mortem brain using a bead based tissue homogenizer (Bertin) and the Trizol procedure (Thermo Fisher Scientific), followed by RNA purification on RNeasy column including a DNAse treatment (Qiagen). RNA Integrity Number (RIN) ranged between 2.9 and 6.9 after extraction, with 18S and 28S rRNA peaks visible. For each sample, a cDNA (NGS) library was prepared with the SMARTer Stranded Total RNA-Seq Kit - Pico Input Mammalian (Takara Bio, kit v.1 for patient 1 and v.2 for patient 2). The libraries of the 2 patients were made independently. The procedure included a random reverse transcription of total RNA into first strand cDNA, a depletion of human ribosomal cDNA, and a final PCR amplification. The cDNA libraries were sequenced independently for the two patients in 1x150bp on a NextSeq500 instrument (Illumina),,using High Output flow cells generating approximately 35 and 116 million reads. A set of reference human transcripts was used as an internal positive control. The inventors have not sequenced the ante-mortem biopsy of Patient #2. The vast majority of raw reads had quality scores (Q scores) above 20. Raw reads were trimmed to remove low quality bases and sequencing adapters at their ends with AlienTrimmer (Criscuolo et al., A, Genomics, 2013, 102, 500-6; version 0.4.0, options -k 10 -m 5 -150 -p 80 -c 012 -q 20). The reads were then assembled with MegaHit (Li et al., Bioinforma Oxf. Engl., 2015, 31, 1674-6; version 1.1.2, with default options). Unassembled reads were kept as singletons. Resulting sequences were aligned after translation in all the six possible reading frames (3 by DNA strand) against a viral protein reference comprehensive database developed by the inventors (RVDB; Bigot et al., F1000Research, 2019, 8, 530) using the DIAMOND program (Buchfink et al., Nat. Methods, 2015, 12, 59-60) . Matches with viral sequences were controlled in NCBI NR protein and NCBI NT databases to identify and eliminate false positive. This led to the identification of 168 sequences (22 contigs and 146 singletons) belonging to the *Peribunyaviridae* family, covering approximately 55% of the closest reference virus genome represented by Umbre virus. No other viral sequence was identified. A search for bacteria or other non-viral pathogen sequences was performed with Kraken2 (Wood et al., Genome Biol., 2014, 15, R46), Centrifuge (Kim D et al., Genome Res [Internet] 2016 [cited 2018 Nov 30]; Available from: http://genome.cshlp.org/content/early/2016/11/16/gr.210641.116) and Methaphlan2 (Truong et al., Nat. Methods., 2015, 12, 902-3) leading to no other viral or bacterial hit. Full CDS of the S, L and M segments were obtained by a combination of RT-PCR and RACE analysis. Patient 1: S segment (SEQ ID NO: 1); M segment (SEQ ID NO: 3); L segment SEQ ID NO: 5). Patient 2: S segment (SEQ ID NO: 90); M segment (SEQ ID NO: 91); L segment SEQ ID NO: 92.

### - Phylogenetic analysis

Alignments of amino acids sequences were done with MAFFT v7.388 (Katoh et al., Nucleic Acids Res., 2002, 30, 3059-66; Katoh et al., Mol. Biol. Evol., 2013, 30, 772-80) and manually adjusted when needed. Identity matrices are given in **Tables I to IV.** RAxML phylogeny with 100 bootstrap replications was performed in Geneious 11.1.5 (Kearse et al., Bioinforma Oxf Engl., 2012, 28, 1647-9) after initial model selection done in MEGA7 (Kumar et al., Mol. Biol. Evol., 2016, 33, 1870-4). Only complete CDS sequences were kept for phylogenetic analysis of the S, M and L segments **(****Figure 4A****, B, C).** The M segment of Koongol virus was removed from the analysis because of the presence of a large deletion (> 300 AA) in the NSn and Gc part of the sequence, which most probably reflects a cell culture passaging artifact as demonstrated for Maguari orthobunyavirus (Pollitt et al., Virology, 2006, 348, 224-32). The M segment of Yacabba virus, and the L segments of Little Sussex virus and Salt Ash virus, were not included in the analysis because of partial CDS sequences. No sequence was available for Little Sussex virus M and S segments. Herbert virus (Family: *Peribunyaviridae;* Genus: ***Herbevirus***) was used as outgroup. **Accession numbers: S segment:** Aino virus (NC_018460.1), Akabane virus (NC_009896.1), Batai virus (JX846604.1), Bunyamwera virus (NC_001927.1), Cache Valley virus (KC436108.1), Caraparu virus (KF254789.1), Chatanga virus (EU479697.1), Herbert herbevirus (JQ659258.1), Iaco virus (JN572067.1), Ilesha virus (KF234073.1), Koongol virus (KP792667.1), Kowanyama virus (KT820204.1), La Crosse virus (NC_004110.1), Leanyer virus (HM627177.1), Macaua virus (JN572070.1), Madrid virus (NC_034498.1), Murrumbidgee virus (NC_022597.1), Oropouche virus (NC_005777.1), Oya virus (JX983192.1), Salt ash virus (KF234258.1), Sathuperi orthobunyavirus (NC_018462.1), Simbu orthobunyavirus (NC_018477.1), Sororoca virus (JN572073.1), Tahyna virus (HM036217.1), Umbre virus (KP792685.2), Wyeomyia orthobunyavirus (JN572082.1), Yacaaba virus (KT820210.1). **M segment:** Aino virus (NC_018459.1), Akabane virus (NC_009895.1), Batai virus (JX846605.1), Bunyamwera virus (NC_001926.1), Cache Valley virus (KC436107.1), Caraparu virus (KF254788.1), Chatanga virus (EU621834.1), Herbert herbevirus (JQ659257.1), Iaco virus (JN572066.1), Ilesha virus (KF234074.1), Kowanyama virus (KT820203.1), La Crosse virus (NC_004109.1), Leanyer virus (HM627176.1), Macaua virus (JN572069.1), Madrid virus (KF254780.1), Murrumbidgee virus (NC_022596.1), Oropouche virus (NC_005775.1), Oya virus (JX983193.1), Salt ash virus (KF234257.1), Sathuperi orthobunyavirus (NC_018466.1), Simbu orthobunyavirus (NC_018478.1), Sororoca virus (JN572072.1), Tahyna virus (HM036218.1), Umbre virus (KP792686.1), Wyeomyia orthobunyavirus (JN572081.1). **L segment:** Aino virus (NC_018465.1), Akabane virus (NC_009894.1), Batai virus (JX846606.1), Bunyamwera virus (NC_001925.1), Cache Valley virus (KC436106.1), Caraparu virus (KF254793.1), Chatanga virus (EU616903.1), Herbert herbevirus (NC_038714.1), Iaco virus (JN572065.1), Ilesha virus (KF234075.1), Koongol virus (KP792669.1), Kowanyama virus (KT820202.1), La Crosse virus (NC_004108.1), Leanyer virus (HM627178.1), Little Sussex virus (KT720483.1), Macaua virus (JN572068.1), Madrid virus (KF254779.1), Murrumbidgee virus (KF234253.1), Oropouche virus (NC_005776.1), Oya virus (JX983194.1), Salt ash virus (KF234256.1), , Schmallenberg virus (NC_043583.1), Simbu orthobunyavirus (HE795108.1), Sororoca virus (JN572071.1), Tahyna virus (HM036219.1), Umbre virus strain IG1424 (KP792687.1), Wyeomyia orthobunyavirus (JN572080.1), Yacaaba virus (KT820208.1).

**Umbre orthobunyavirus strain Moissiacense (Moissiacense virus):** S segment (SEQ ID NO: 2); M segment (SEQ ID NO: 4); L segment SEQ ID NO: 6.

**Umbre orthobunyavirus strain Marna** : S segment (SEQ ID NO: 90); M segment (SEQ ID NO: 91); L segment SEQ ID NO: 92.

### - Neuropathology

The biopsy sample was left overnight in formalin (10% of the commercial formalin solution containing 4% formaldehyde). After autopsy, fresh brain slices were stored frozen at -80°C prior to nucleic acid extraction. The whole brains were immersion-fixed in formalin 10%. In case 1, only 9 samples were available. In case 2, a systematic protocol was applied. Samples of cerebral neocortex and hippocampus, basal ganglia, brainstem, cerebellum and spinal cord were analyzed.

### - Immunohistochemistry

The samples were paraffin-embedded. Three µm thick sections were obtained and stained with haematein-eosin, Periodic Acid Schiff (PAS) and Luxol Fast Blue. t. Immunochemistry was performed with various primary antibodies in a Ventana Ultra stainer (Roche) with diaminobenzidine as a brown chromogen. Primary antibodies included a rabbit monoclonal anti-CD3 (clone 2GV6, prediluted, Roche), a mouse monoclonal anti-CD163 (clone MRQ-26, prediluted, Roche) and a polyclonal rabbit anti IBA1 (dilution 1/500, Wako), all incubated 32 min at 37°C. Immunohistochemistry was performed independently for the two patients.

### - Western Blot

The search for PrPres was performed in the two cases by Western blot according to published protocols (Lasmézas et al., Science, 1997; 275:402-405).

### - In-situ hybridization

ViewRNA ISH Tissue Assay Kit 2-plex (Thermo Fisher Scientific) was used to detect RNA sequences. According to the manufacturer, the technique has a sensitivity of one single copy and is based on branched DNA technology. ViewRNA Probes have a double Z configuration: one side of the Z is complementary to the target sequence and its other side is complementary to the pre-amplifier DNA sequence. A cocktail of 95 custom bDNA probes was designed to target the 3 genomic segments of Umbre orthobunyavirus strain Moissiacense (Moissiacense virus) (15 probes for segment S, 40 probes for segment M, 40 probes for segment L) and was revealed (as the "probe type 1" of the kit) by a red signal. A mix of control probes targeting human GAPDH, ACTB and PPI transcripts was revealed (as the "probe type 6" of the kit) by a blue signal. To unmask RNA targets, the tissue sections (after deparaffinization by xylene and ethanol) were pretreated by heating at 95°C during 20 min in PBS 1X, cooling at room temperature (RT) during 5 min, washing 2 x 1 min in distilled water, washing 1 min in PBS 1X, protease digestion during 10 min at 40°C in a hybridizer Denaturation & Hybridization System (NB-12-0001, Neobrite), and washing 2 x 1 minute in PBS at room temperature (RT). Tissue sections were fixed 5 min in formaldehyde 4% at RT, then washed 2 x 1 min in PBS 1X. Probes diluted in Probe Set Diluant QT (ThermoFisher) - a proprietary aqueous solution containing formamide, detergent, and blocker- were added on the tissue section and were hybridized during 2 hours at 40°C in the hybridizer. The sections were washed 3 x 2 min in Wash Buffer. During the amplification step, sections were incubated 25 min at 40°C in PreAmplifier Mix containing the PreAmplifier DNA type 1 and type 6, washed 3 x 2 min in Wash Buffer, incubated 15 min at 40°C in Amplifier Mix containing the Amplifier DNA type 1 and type 6, and washed 3 x 2 min. In order to produce the blue signals, the section were incubated with 6-AP (an alkaline phosphatase binding the amplifier DNA type 6) during 15 min at 40°C in the hybridizer, washed 3 x 3 min in Wash Buffer, incubated with Fast Blue solution during 30 min at RT, and washed 3 x 3 min in Wash Buffer. The 6-AP was quenched by AP STOP QT during 30 min at RT. In order to produce the red signals, the section were incubated with 1-AP (an alkaline phosphatase binding the amplifier DNA type 1) during 15 min at 40°C in the hybridizer, washed 3 x 3 min in Wash Buffer, incubated with Fast Red solution during 45 min at 40°C in the hybridizer, and washed 1 min in PBS. Haematoxylin was used for counterstaining. *In-situ* hybridization was performed at different times for the two patients, with Patient #1 having secondarily served as a positive control for Patient #2. The five negative controls used for ISH were other encephalitis cases coming from the larger series of brain samples (see section "Context of the study") that have been sequenced with a comparable sequencing depth and that were either negative (3/5) or positive for viruses other than Umbre (2/5).

### - Mosquito capture

Approximately 4,000 *Culex pipiens* female mosquitoes were captured in 2015 in the French Rhône Delta region as described in Eiden et al. 2018 (Eiden M, Gil P, Ziegler U, et al. Infect Genet Evol J Mol Epidemiol Evol Genet Infect Dis 2018;61:151-4) and 133 pools of approximately 30 individuals were made. Mosquito capture was carried for a 24-hour period once a week between June and September. Captures were carried out in the 6 sites in parallel the same days. The June-September period covers most of the mosquito season in the study region. The population of Culex pipiens is generally rather low before or after this period (usually less than 10 females per catch). In addition, this period was selected because it includes the population peak for Culex pipiens (usually June-July depending on the site) and the peak of prevalence in mosquitoes and clinical cases in vertebrates for Culex-borne arboviruses in the region (i.e. September for West Nile and Usutu viruses, e.g. Eiden et al. 2018, precited). 133 pools of approximately 30 individuals were made. In addition, around 100 *Culex pipiens,* 30 *Culex Sp.,* 100 *Aedes caspius,* 50 *Aedes detritus,* 10 *Aedes vexans,* 10 *Aedes Sp.,* and 20 *Anopheles* mosquitoes were captured in 2017 in Camargue and Languedoc (Southern France) and species-specific pools of 5-20 individuals were made. Of the mosquitoes captured in 2017, no positivity was recorded.

### - RT-PCR

RT-PCRs were done on the exact same tissue RNA extracts that were used for sequencing. Reverse transcription reactions were performed using the SuperScript IV First-Strand Synthesis System (Thermo Fischer Scientific). Quantitative PCR were done in SYBR Green format with 45 cycles of amplification. Human beta-actin was included in the RT-PCR experiments as a positive control of the cDNA. No Template Control (NTC) wells, validating the absence of cDNA or DNA contaminant into the PCR mix, were also added to the experiment. Positive amplicons after 45 cycles were purified on gel and serial-dilutions were made to generate standard curves. Primer pairs used for detection of Moissiacense virus were: L segment AGAATTGGTTATCCCAGATGAGGT (forward; SEQ ID NO: 14) and GCCATAAATTGAAAATGGTTCTCCA (reverse; SEQ ID NO: 15); M segment ACAGGRCAAATAGCACTAAAGGT (forward; SEQ ID NO: 12) and CCTTCRTCTCTCACTTTGCAG (reverse; SEQ ID NO: 13); S segment AAACGCAGAACTGGGTAGCA (forward; SEQ ID NO: 10) and GAGTTAGCTCATCGTCCGCA (reverse; SEQ ID NO: 11). The primer pair used for detection of the Koongol virus L segment was GACCCAATACTGTAAACAG (forward; SEQ ID NO: 69) and CGTCAGAATGCACCATTG (reverse; SEQ ID NO: 70). PCR performed after omitting the reverse transcription step for samples C2, F2, G2 and C9 were negative, supporting the detection of viral RNA rather than endogenous viral DNA in mosquitoes.

### - CSF analysis

RNA extracts from 193 CSFs collected in patients (attending both University hospitals of Montpellier and Nimes, two towns close to the Camargue region) presenting with a meningitis or meningo-encephalitis during the course of a regional arbovirus surveillance program during the peak mosquito activity period from 1^{st} May to 31 November 2016 and 2017 were tested by RT-PCR using primers specific for the S segment of Moissiacense virus (Umbre virus). These 193 CSFs have not been analyzed with the serological assay due to insufficient quantity remaining after RT-PCR.

### - Serology

### Recombinant protein preparation

### - Expression of the Gc head domain in eukaryotic system

The Gc head domains of Umbre orthobunyavirus strain Moissiacense (residues G477-D730 of the polyprotein precursor; SEQ ID NO: 64) and Schmallenberg virus (residues Q465-I702 of the polyprotein precursor (GenBank accession number: CCF55030.1) carrying a C-terminal Strep-tag (GGWSHPQFEK; SEQ ID NO: 97) were produced in recombinant Drosophila S2 cells (Gibco) grown at 28°C in HyClone SFM4Insect medium with L-glutamine (GE Healthcare) supplemented with 25 U/mL penicillin/streptomycin (Gibco). For this purpose, a codon-optimized synthetic gene fragment (Invitrogen) was cloned into the pMT expression vector (Invitrogen) downstream of a BiP secretion signal. The expression plasmid was co-transfected with the selection plasmid pCoPURO (Iwaki et al., BioTechniques 2003; 35:482-484, 486) at a mass ratio of 20:1 using the Effectene transfection reagent (Qiagen) according to the manufacturer's instructions. A polyclonal stable S2 cell line was established by selection with 7.5 µg/mL puromycin (Invivogen), which was added to the medium starting 40 h after transfection. The culture was expanded to 1 L of 10⁷ cells/mL in Erlenmeyer flasks shaking at 100 rpm and at 28°C. Recombinant protein expression was then induced with 500 µM CuSO₄. The cell supernatant was harvested 1 week after induction, concentrated to 50 mL on a 10 kDa MWCO PES membrane (Sartorius), pH-adjusted with 0.1M Tris-Cl pH 8.0, cleared from biotin with 15 µg/mL avidin, cleared from precipitate by centrifugation at 4000 x g for 15 min at 8°C, and was used for affinity purification on a 5 mL Strep-Tactin Superflow he column (iba Life Science). The protein was further purified by gel permeation chromatography on a HiLoad Superdex 200 pg column (GE Healthcare) in 20 mM Tris-Cl pH 8.0, 150 mM NaCl. Aliquots were flash-frozen on liquid nitrogen and stored at -80°C.

### - Expression of then protein in prokaryotic system

The N protein (SEQ ID NO: 2) was expressed from a codon-optimized synthetic gene in E. coli BL21 with N-terminal Strep- and His-tags. The protein was sequentially purified on a HisTrap column (GE Healthcare), a StrepTactin column (iba) and a Superdex S200 column (GE Healthcare). Peak fractions in 20 mM Tris-Cl pH 8, 150 mM NaCl were frozen.

### Serum samples

Three hundred serum samples randomly collected from patients attending the Montpellier University hospital as part of a collection for seroepidemiological investigation through the Arbosud project (Montpellier University MUSE project) were screened. Additional 34 serum samples from patients with diagnosis of meningitis or meningo-encephalitis were also tested (Institutional review board 2019-IRB-MPT-05-06).

### ELISA serological assay

Two enzyme linked immuno assays were set up with two recombinant antigens, corresponding to the head domain of the Gc protein for respectively the Umbre virus Moissiacence strain and the Schmallenberg virus used as a negative control. Reactivity of every serum samples was concomitantly tested against each antigen in 96 wells plates. Plates were coated overnight at 4°C with 100µL of 2 µg/mL of the respective antigens. Plates were subsequently washed 3 times with PBS-tween 0.1% and further blocked with PBS-tween 0.01% plus 2% of bovine serum albumin during 2 hours at room temperature. Plates were washed 3 times before 100 µL of a one hundred dilution of serum samples in blocking solution was added. Serum samples were incubated 3 hours at room temperature. Horseradish peroxidase secondary antibody diluted in blocking solution was added after 3 washes and incubated one hour at 37°C, under agitation. TMB substrate was added and plates remained in dark at room temperature for 20 min before reaction was further stopped with H₂SO₄. The 450nm optical densities were recorded for each sample. Samples giving the highest values were alternatively tested through a commercial pan-species ELISA Schmallenberg virus assay that displays the N protein as targeted antigen, following the manufacturer recommendations (ID Screen Schmallenberg virus indirect Multi-species, IDvet, France).

### LIPS serological assay

The search for antibodies against Umbre virus are carried out on sera using a Luciferase Immuno-Precipitation System (LIPS) assay as described (Temmam et al., Front Microbiol., 2019, 10, 2315, doi.org/10.3389/fmicb.2019.02315) using the full Gc protein (SEQ ID NO: 60) or the Gc extracellular domain (SEQ ID NO: 61) or the N-terminal Alpha helix (SEQ ID NO: 62) or the N protein (SEQ ID NO: 2). Viral antigens are produced in HEK-293F cells transfected with plasmids expressing the gene for Nanoluc fused to the C-terminus of the viral protein. Recombinant proteins are harvested from the supernatant or cell lysate without any purification step, and incubated with 10 µL of animal serum. The immune complexes are precipitated with protein A/G-coated beads, washed, and the luciferase activity is monitored for example on a Centro XS³ LB 960 luminometer (Berthold Technologies, France). The positivity threshold can be defined as the mean of 10 negative controls (without serum) + five standard deviations.

### Serological survey

The Gc head domain of Schmallenberg virus, an orthobunyavirus of ruminants belonging to the Simbu serogroup, served as a negative control. It was not possible to use the sera of the two Umbre patients as a positive control because of their immunoglobulin deficiency. A positive control was used for the Schmallenberg virus antigen and was tested undiluted and at a 10-fold dilution. Three hundred sera from control patients (the control group) and 34 sera from encephalitis cases (the encephalitis group) were screened. In the control group (N=300), optical densities ranged from 0 to 4 for both antigens **(****Figure 7****).** Because no human cases of Schmallenberg virus infection have been reported (Ducomble et al., Emerg Infect Dis, 2012, 18:1333-1335; Reusken et al., Emerg Infect Dis 2012; 18:1746-1754.) the inventors verified and confirmed the absence of detectable antibodies against the immunodominant nucleocapsid N protein of Schmallenberg virus for samples with the highest densities, using a reference veterinary kit which included a positive control (ID Screen Schmallenberg virus indirect Multi-species, IDvet, France). Since the Gc head domains of Schmallenberg virus and Umbre virus were designed in an orthologous manner and produced under strictly identical conditions, the inventors concluded that optical densities values up to 4 corresponded to background noise. In the encephalitis group (N=34), all optical values were inferior to 3 **(****Figure 8****).** The observed statistical differences were therefore attributed to random background noise and it was concluded that no specific antibody response was recorded.

### 2. Case report

### Context of the study

The two cases presented in this study were selected from a larger series of sixty brain samples collected either from autopsy or biopsy, with brain inflammation, in which the inventors performed a molecular screening for the presence of pathogens. Over a period of 2 years, seventeen of these samples were analyzed by high-throughput sequencing with a metatranscriptomics approach. Patient #2 had undergone a brain biopsy followed 10 days later by a diagnostic autopsy. Patient #1 had been included in the National Creutzfeldt-Jakob disease (CJD) surveillance network, coordinated by the neuropathology department of Pitié-Salpêtrière. This network collects the postmortem samples from all cases in France with a diagnosis of "possible" or "likely" CJD, according to the World Health Organization criteria (WHO Recommended surveillance standards WHO/CDS/CSR/99.2). The aim of this network is not only to confirm CJD but also to identify differential diagnoses in cases of rapidly progressive neurological diseases. In a large series of 1572 cases autopsied between 1992 and 2009, the inventors found that 30 % of the collected cases were not prion diseases (Peckeu L et al., Euro Surveill Bull Eur Sur Mal Transm Eur Commun Dis Bull, 2017; 22).

### Case 1

A 21-year-old man was was admitted to the neurology department of Toulouse Hospital in October 2013 for swallowing impairment and fluctuation of consciousness. He was living in Moissac, in the south-west of France. He had developed behavioral changes suggestive of depression after a party during which he could have used cocaine and cannabis. His psychiatrist had prescribed selective serotonin reuptake inhibitors. The clinical state had not improved. He was then referred to the psychiatry department at Toulouse University Hospital. His past condition included Bruton's agammaglobulinemia (Omim # 300755), an X-linked immunodeficiency characterized by the absence of circulating B cells and low to undetectable levels of all immunoglobulin isotypes in the serum. He was treated since early childhood by repeated infusion of intravenous immunoglobulins together with an anti-infectious prophylaxis by valaciclovir and sulfamethoxazole/trimethoprim. He also suffered from Crohn's disease, which was treated with mesalazine and azathioprine.

The patient was anorectic and had lost 10 kg during the last weeks. He was afebrile. The diagnosis of catatonia was considered. He did not respond to antidepressants, benzodiazepines and neuroleptics at high doses. Upon admission in the Neurology department, brain MRI, with FLAIR (FLuid-Attenuated Inversion Recovery) sequence and diffusion-weighted imaging, showed a diffuse hypersignal in the insular, posterior cingulate and occipital cortices, and in the caudate nucleus and putamen, bilaterally **(****Figure 1****, a & b).** FDG PET/CT scan showed a diffuse cortical hypofixation, more severe in the left hemisphere and no hypermetabolism outside the brain. Blood cell count showed a decreased number of lymphocytes (500 G/L). There was no anti-thyroglobulin and anti-thyroperoxidase antibody in the serum. Serological tests for *Borrelia burgdorferi, Treponema pallidum,* polyomavirus, HIV, and hepatitis B, C, and E viruses were negative - a negativity that was difficult to interpret in a patient with agammaglobulinemia. The concentration of serum C-reactive protein was increased (150 mg/L; normal value below 6 mg/L). The blood levels of aspartate aminotransferase, and alanine aminotransferase were increased two folds.

Total proteins concentration, glucose level, and cell count were normal in the cerebrospinal fluid (CSF). No oligoclonal bands were detected. CSF PCRs for enteroviruses, herpesviruses, and *Tropheryma whipplei* were negative. The search for Ri/La/Hu, VGCC, NMDA receptor, GABA, ANNA-1, ANNA-2, gangliosides, AQP4, and MOG antibodies was negative. The CSF was positive for the 14-3-3 protein.

The patient died two months and a half after the first symptoms. The rapid course of the disease, the cortical and striatal hyperintensity at MRI, the positivity of 14-3-3 protein in the CSF, and a history of repeated treatment with blood products had raised the diagnostic hypothesis of Creutzfeldt-Jakob disease, either iatrogenic or variant. The diagnosis of encephalitis, by contrast, was thought implausible: the patient had been afebrile during the whole course of the disease and the CSF cell count was normal. The autopsy was limited to the brain. The brain samples were referred to the national neuropathology network for the surveillance of Creutzfeldt-Jakob disease.

### Case 2

A 58-year-old woman was referred to the emergency department of Paris Pitié-Salpêtrière Hospital in October 2018 for anorexia and psychomotor slowing, without fever. She had been followed in the immunology department (Saint Louis Hospital, Paris) for a complex immunologic disorder. The diagnosis of a cutaneous follicular lymphoma had been made 17 years earlier. She had been treated with radiotherapy, surgery and chemotherapy (rituximab-cyclophosphamide-vincristin-prednisone, then rituximab-cyclophosphamide). She had been in complete remission for 9 years. She had developed a humoral immune deficiency characterized by low IgG and undetectable IgM levels in the absence of circulating B cells. She had recurrent bacterial infections for which she had received polyvalent immunoglobulins. She was also suffering from vitiligo and lichen planus. She had been followed for more than 10 years for a severe autoimmune hepatitis requiring immunosuppressive treatment with sirolimus and leading to cirrhosis, for which transplantation was being considered. At admission in the intensive care unit, psychomotor slowing was marked. There were no focal neurological signs. CSF was clear and contained 10 white blood cells/mm³ (N<5), with CD20 negative lymphocytes at various stages of activation and some macrophages. CSF glucose was normal (3.53 mmole/L). The protein level was increased (0.67 g/L, N< 0.40). MRI was normal **(****Figure 1****, c-e).** Three months later, the patient was apathetic. The CSF contained 34 white blood cells/mm3 and 4,200 red blood cells/mm3. CSF protein level was 0.47 g/L. Interferon alpha was increased in the CSF (6 kUI/L, N<2), normal in the serum (2 kUI/L). The search for autoantibodies (including NMDAr, Yo, RI, Hu, amphiphysine, CV2, Sox1, GAD, Ma1, Ma2) was negative. CSF PCRs for viruses (EBV, CMV, HSV1 and 2, VZV, HHV6, HHV8, adenovirus, enterovirus, polyomavirus) were all negative. She received intravenous immunoglobulins in the hypothesis of dysimmune encephalitis. Four months later, her neurologic condition worsened. She had lost her balance, had fallen several times and was mute. She was transferred to the neurology department at Pitié-Salpêtrière Hospital. She lived in Paris area (Val-de-Marne). Her relatives indicated that she had traveled extensively, visiting China, Japan, Australia, Spain, Israel and Italy in previous years. They also reported that a few weeks before the onset of the symptoms, she had sojourned in Camargue, south of France, and went on a 10-day Mediterranean cruise. Despite treatment with amoxicillin and aciclovir, she progressed to akinetic mutism within three months. Hyperintensity in the hippocampus, the temporal pole and the insula were observed bilaterally and progressed to atrophy **(****Figure 1****, f-h).** The spinal MRI was normal. Repeated EEG showed slow waves, with pseudo-periodic patterns, similar to those observed in Creutzfeldt-Jakob disease. She became comatose, despite Ig replacement therapy, high dose steroids pulses, and plasma exchange in the hypothesis of autoimmune encephalitis. It was decided to perform a biopsy of the right frontal middle gyrus in the absence of focal lesion visible at MRI. Nine days after the biopsy, the patient developed acute hepatic failure due to portal venous thrombosis. She died after an evolution of five months and a half. The diagnosis of encephalitis was suspected but its cause remained undetermined. An autopsy was performed.

### 3. Results

### - Neuropathology

### Case 1

The brain weighed 1200 g. Its external aspect was unremarkable. Microscopic examination showed severe neuronal loss and spongiosis in the cerebral cortex and in the striatum in both hemispheres **(**Figure 2, d); the number of Purkinje cells was also severely reduced. The gliosis was marked, made of fibrillar astrocytes with abundant eosinophilic cytoplasm. Microglia -IBA1, CD163 and CD68 positive- were diffusely activated **(****Figure 2****, a-c**). Numerous microglial nodules were observed, in particular in the pyramidal layer of the hippocampus and in the dentate nucleus of the cerebellum **(****Figure 2****, a & b).** The nodules sometimes contained a neuron (neuronophagia) **(**Figure 2, a). A few T lymphocytes, CD3 and CD8 positive, were diffusely distributed around the vessels and in the parenchyma **(**Figure 2, e). CD20 and CD4 immunohistochemistry was negative **(**Figure 2, d & e). No viral inclusion was observed. Luxol Fast Blue staining did not show significant demyelination. PrP 12F10 immunohistochemistry was negative as well as the PrPres Western blot.

### Case 2 biopsy

The biopsy sample consisted of 1 cm³ of cerebral cortex comprising leptomeninges, gray and white matter. Microscopic examination showed marked astrogliosis and severe microglial activation, confirmed by CD163 immunochemistry. Some perivascular cuffs were observed, made of CD3 positive, small T-lymphocytes, 80% of them being CD4- and 20%, CD8-positive. There was no granuloma nor multinucleated giant cell.

### Case 2 autopsy

The brain weighed 1055 g. Gross examination showed thinning and softening of the temporal poles, and atrophy of the caudate nucleus and cerebral peduncles, bilaterally. An inflammatory infiltrate of low abundance, made of small CD3 positive T lymphocytes, was observed in the leptomeninges and around the veins in the caudate nucleus and mesencephalon. The arterial walls were not altered; there was no evidence for vasculitis. Severe neuronal loss, astrogliosis, and diffuse microglial activation were observed in the neocortex, hippocampus, striatum and cerebellum on both sides **(****Figure 3****, a & b).** There was a severe loss of pyramidal neurons in CA1 to CA4 sectors **(**Figure 3, d). Neurons were replaced by reactive astrocytes with enlarged cytoplasms. The numerical density of the granule neurons of the dentate gyrus was decreased. CD163 immunohistochemistry showed massive microglial activation in the pyramidal sectors of the hippocampus and in the dentate gyrus **(**Figure 3, a). In the cerebellum **(**Figure 3, b), neuronal loss and microglial activation were severe in the dentate nucleus, as well as in the granular and Purkinje cell layers. The loss of Purkinje cells was complete, underlined by the hypertrophy of Bergmann glia. The neuronal loss was severe in the *substantia nigra, locus cœruleus* and pontine nuclei. Numerous macrophages were observed in the root of the oculomotor nerve in which axonal and myelin loss was severe **(**Figure 3, e). Nodules of neuronophagia were found in the medulla oblongata and in the anterior horns of the spinal cord **(**Figure 3, c). The spinal roots were spared.

### - Identification, characterization and quantification of Umbre virus sequences from patients sample

*Post-mortem,* metatranscriptomics identified viral sequences related to Umbre virus in the brain of patient 1 and in the brain and spinal cord of patient 2. Full coding sequence of the S, L and M segments were obtained by a combination of RT-PCR and Rapid Amplification of cDNA Ends (RACE). Patient 1: SEQ ID NO: 5 (L segment), SEQ ID NO: 3 (M segment) and SEQ ID NO: 1 (S segment). Patient 1: SEQ ID NO: 92 (L segment), SEQ ID NO: 91 (M segment) and SEQ ID NO: 90 (S segment). This novel virus, named Moissiacense virus in reference to the French region where patient 1 was living, is a member of a currently unnamed clade or serogroup **(****Figure 4A****, B, C),** and is closely related to Umbre virus strain IG1424, isolated in India.(97.6% amino acid sequence identity (AA Id) and 88% nucleotide sequence identity (nt Id) for L segment; 94.3% AA Id and 86% nt Id for M segment; 97.5% AA Id and 92% nt Id for S segment) and to a lesser extend to Koongol virus (70.5%- AA Id and 68% nt Id for L segment; 55.0% AA Id and 66% nt Id for S segment; no data for M segment) **(Tables I to III).**

The viral sequences found in the two patients were closely related to Umbre virus strain IG1424, isolated in India (97.6%-97.9% amino-acids identity for the L segment), and to a lesser extent to Koongol virus, isolated in Australia (70.5%-71.0% AA Id for L segment) **(****Figure 4C****; Tables III and IV).** According to the species demarcation criteria of the International Committee on Taxonomy of Viruses (ICTV) for orthobunyaviruses (International Committee on Taxonomy of Viruses (ICTV). Available at: https://talk.ictvonline.org/ictv-reports/ictv 9th report/), the virus strain IG1424 and the two viral strains identified from the two patients belong to the same species and represent different strains of Umbre virus. The inventors therefore gave the names Umbre orthobunyavirus strain Moissiacense (Patient 1) and Umbre orthobunyavirus strain Marna (Patient 2), in reference to the French regions where the patients lived (Moissac and Val-de-Marne, respectively). Moissiacense virus and Marna virus also appears to be very closely related to Little Sussex virus (100.0% AA Id for the 96 AA PCR amplicon on L segment)

### (Table IV).

PCR primer pairs were designed for the specific detection of Moissiacense virus, Umbre virus and Little Sussex virus (Moissiacense virus species-specific primers): SEQ ID NO: 10-11 (S segment); SEQ ID NO: 12-13 (M segment): SEQ ID NO: 14-15 (L segment). Umbre virus S, L and M segments were detected by RT-PCR on the patient's brain sample using the Moissiacense virus species-specific primers **(****Figure 5****).** *C. pipiens* samples containing related virus sequences were negative by PCR with the Moissiacense virus species-specific primer. These results demonstrate the specificity of the designed PCR primers for Moissiacense virus species (Moissiacense virus, Umbre virus and Little Sussex virus). Determination of viral titer in the patient's brain sample by RT-qPCR indicated approximately 10⁶ to 10⁸ genome copies/gram of tissue for both patients. Patient 2 also tested positive, albeit less strongly, in the liver and lymph nodes with viral genome copies ranging from 10⁴ to 10⁶ per gram of tissue.

No CSF from patient 1 was available. Three CSF samples from patient 2, collected less than three months before death, were tested by RT-PCR for the three segments of Moissiacense virus species (Umbre virus) and were negative. In addition, 193 CSFs from meningo-encephalitis cases collected in Montpellier and Nimes, two southern cities close to the Camargue region were tested by RT-PCR, for the Moissiacense virus species (Umbre virus) S segment. None was positive.

### - Umbre virus infects brain neurons

In the absence of commercially available antibody specific to the Moissiacense group of viruses, *in-situ* hybridization probes were designed to detect the S, M and L segments of Moissiacense virus. *In situ* hybridization (ISH) was performed on tissue sections from samples of frontal cortex, in which microglial activation was severe. Probes homologous to the S, M and L segments of Umbre virus were positive in the cell body and the apical neurite of pyramidal neurons **(**Figure 2, f and Figure 3, f), while glial cells, macrophages or lymphocytes were negative. Positive neurons were scattered in case 1 but numerous in case 2. Viral sequences were not found in five biopsy and autopsy samples of control cases with encephalitis of other documented causes. These data indicate that Umbre virus infects and replicates in cortical neurons.

### - Detection and prevalence of viral sequences related to Umbre virus in Culex pipiens captured in France

Umbre virus has never been described in Europe. Since this virus is borne by mosquitoes (Dandawate et al., Indian J Med Res, 1969; 57:1420-1426) the inventors attempted to determine if it could be detected in mosquitoes captured in Camargue between 2015 and 2017 (see Material and Methods). Camargue is located between the Mediterranean Sea and the two arms of the Rhône delta. It is a touristic area, in the South of France, inhabited by many species of insects and notoriously by mosquitoes. Patient 2 stayed in Camargue during the weeks preceding the onset of her disease. Patient 1 was living 200 km west of the Rhone delta and had never travelled outside continental France.

All 133 pools of *Culex pipiens* mosquitoes captured in 2015 in Camargue were negative for PCRs targeting the S, M and L segments of Moissiacense virus. However, 4 out of the 133 pools were positive for Koongol L-segment by RT-PCR (samples C2, C9, F2 and G2). PCR performed after omitting the reverse transcription step was negative, supporting the detection of viral RNA rather than endogenous viral DNA. Sanger sequence analysis of the viral amplicons from mosquitoes confirmed the proximity between Koongol virus (82.1-83.2% AA Id), Little Sussex, Umbre and Moissiacense viruses (75.8-76.8% AA Id) **(****Figure 4D** **and Table IV).** Of the mosquitoes captured in 2017, none were positive, which might reflect the lower number of mosquitoes tested. Based on the number of positive pools and the size of the pools from the 2015 captures, the prevalence of Koongol-like sequences in *Culex pipiens* was estimated by statistical prediction models to be approximately 0.1% (variances ranging from 10⁻⁷ to 10⁻³ depending on the model used), assuming either unknown or perfect sensitivity and specificity values of the PCR (Cowling et al., Prev. Vet. Med., 1999, 39, 211-25).

### - Serological survey

Three hundred sera from control patients and 34 sera from encephalitis cases from the South of France including the Camargue region were screened with an ELISA test that the inventors developed to detect Umbre virus antibodies **(****Figures 7-8****).** No serum gave a positive result.

### 4. Discussion

Inaccurate diagnosis of encephalitis is a main issue as immunosuppressive treatments can be deleterious in case of viral infection. Metatranscriptomics identified sequences of Umbre virus in brain samples from two immunocompromised patients with clinical and pathological signs of encephalitis. In the two cases, behavioral symptoms preceded neurological signs caused by involvement of the basal ganglia (extrapyramidal syndrome) and of the brainstem (swallowing difficulties). There was no fever. The CSF was normal or with a mild hypercytosis. Pathologically, microglial activation, perivascular cuffing by mononucleated cells including T-lymphocytes, neuronal loss and neuronophagia ascertained the diagnosis of encephalitis. The relative scarcity of lymphocytes, contrasting with the abundance of microglial activation, and the frequency of neuronophagia were noticeable in the two cases. The changes predominated in the grey matter (cerebral cortex, striatum, cerebellar cortex and dentate nucleus). At least in case 2, there was a severe involvement of the brainstem and spinal cord. Although never isolated previously in cases of human or animal encephalitis, Umbre virus may be taken as causal in the two reported cases because of the high number of recovered genomes (around 10⁸ genome copies/gram) and the demonstration that Umbre virus infected the neurons of the two patients as seen by *in situ* hybridization, a key feature of arboviruses responsible for encephalitis (Salimi et al., Neurother J Am Soc Exp Neurother, 2016, 13:514-534.) In addition, no other pathogen was found.

The work is original because it deals with the damaged brain tissue obtained from a brain biopsy or autopsy and analyzed by confirmed neuropathologists and the analysis is done without a priori, by methods of very deep sequencing (High Troughput sequencing or NGS), followed by a comparison of the sequences with viral and general nucleotide and protein data bases. The originality of the work is strengthened by the fact that PCR consensus assays targeting a broad range of orthobunyaviruses (Lambert AJ, Lanciotti RS, J. Clin. Microbiol., 2009, 47, 2398-404) would have missed Moissiacense virus due to sequence variation **(****Figure 6****).**

Within the genus *Orthobunyavirus,* Umbre virus, Little Sussex virus and Koongol virus belong to the Koongol group of viruses, a group that is yet to be classified by the ICTV but that is distinct from other *Orthobunyavirus* groups such as the California encephalitis, Bunyamwera, Simbu and Wyeomyia groups. Within the order *Bunyavirales,* members of the California serogroup (genus *Orthobunyavirus,* family *Peribunyaviridae)* and the Rift Valley Fever virus (genus *Phlebovirus,* family *Phenuiviridae*) can be responsible for human encephalitis. Regarding viruses related to the Koongol group, however, their pathogenic potential remains unknown.

Based on the species demarcation criteria of the International Committee on Taxonomy of Viruses (ICTV) for orthobunyaviruses, Moissiacense virus, Umbre virus and Little Sussex virus would belong to the same species (>90% identical AA identity; ICTV report, 30 Nov 2018 (available from: https://talk.ictvonline.org/ictv-reports/ictv 9th report/). Previously, Umbre, Little Sussex and Koongol viruses have been identified in *Culex sp.* in India, Australia and New Guinea (Dandawate et al., Indian J. Med. Res., 1969, 57, 1420-6; Doherty et al., Aust. J. Exp. Biol. Med. Sci., 1979, 57, 509-20; Doherty et al., Aust. J. Exp. Biol. Med. Sci., 1963, 41, 17-39; Karabatsos, edited by N. International catalogue of arboviruses, including certain other viruses of vertebrates [Internet]. 3rd ed. San Antonio, Texas: American Society of Tropical Medicine and Hygiene for The Subcommittee on Information Exchange of the American Committee on Arthropod-borne Viruses; 1985. Available from: https://catalyst.library.jhu.edu/catalog/bib 66192 ).

Umbre virus (genus *Orthobunyavirus,* family *Peribunyaviridae)* was first identified in *Culex sp.* mosquitoes in India in the 1950s and was catalogued as an arthropod borne (arbo-) virus after successful disease transmission experiments in mice (Dandawate et al., Indian J Med Res 1969; 57:1420-1426). In the 1970s, related viruses named Little Sussex and Koongol were identified in *Culex sp.* in Queensland, Australia (Doherty et al., Aust J Exp Biol Med Sci 1979; 57:509-520; Doherty et al., Aust J Exp Biol Med Sci 1963; 41:17-39.). The pathogenic potential, in human, of viruses related to the Umbre/Koongol group, however, remained uncertain despite that they are classified by the Center for Disease Control and Prevention (CDC) as 'Probable Arbovirus'. Serological surveys based on hemagglutination assay have suggested that members of the Umbre/Koongol group could infect several mammals, but this has not been confirmed with a more specific technique such as seroneutralization (Shchetinin et al., Viruses, 2015; 7:5987-6008). Only recently, a partially characterized orthobunyavirus closely related to Umbre virus, causing severe kidney disease in broiler chickens, has been reported in Malaysia (Palya et al., Emerg Infect Dis., 2019; 25:1110-1117). The inventors now report the first human cases leading to brain infection with a virus from the Umbre/Koongol group.

In this study, the origin of the two Umbre virus strains could not be elucidated. No Umbre virus could be traced by RT-PCR in pools of mosquitoes isolated in Camargue. Sequences from Koongol virus could however be identified in a small number of mosquitoes, indicating the presence of still unidentified orthobunyaviruses, close to Umbre virus, in the south of France. The inventors concluded that the two patients were possibly infected by mosquito bites - a finding that should stimulate more comprehensive epidemiological studies of the virome of mosquitoes in this area.

Diagnosis of orthobunyavirus encephalitis is based on antibody testing (Miller A et al., Hosp. Pediatr., 2012, 2, 235-242). The inventors have tested the prevalence of antibodies binding the Gc head domain protein of Umbre virus in a first series of control (N=300) and encephalitis (N=34) cases but did not find any positive case. It is noteworthy that, because of the hypo- or agammaglobulinemia status of the two patients, the absence of human serum that can serve as a positive control complicated the methodology. The observations of the inventors nevertheless suggest that the prevalence of infection in the general population is low or that the virus is unable to infect most individuals with a normal immune system. The actual risk of disease transmission in immunocompromised individuals may be different and remains unknown. Immune deficit - and particularly the deficit in neutralizing antibodies (Hellert et al., Nat Commun 2019; 10:879) - may have played a crucial role in the development of the encephalitis in a way that remains to be investigated.

As for other major arboviruses, like JEV, Umbre virus seems to be hardly detectable in the CSF after the onset of the disease (Dubot-Pérès et al., Lancet Infect. Dis., 2015, 15, 1376-7; Touch et al., Trop. Med. Int. Health TM IH, 2009, 14, 1365-73). (three CSFs from P2 tested negative, and none of the 193 CSFs taken from suspected cases of meningo-encephalitis in two southern cities close to the Camargue region tested positive). Similarly, negative PCR-testing of the CSF was also recorded for Cache Valley virus, another orthobunyavirus identified recently in the brain of an immunodeficient patient (Wilson et al., Ann. Neurol., 2017, 82, 105-14). This suggests that in immunodeficient patients, especially patients without circulating B cells, persistent neurological symptoms should evoke a viral encephalitis that only a brain biopsy can confirm at an early stage.

In conclusion, the convergence of virological and epidemiological data shows that the inventors have identified Umbre virus as a new neurotropic arbovirus in Europe, belonging to the orthobunyaviruses. Another close species was identified in *Culex pipiens* mosquitoes from the same region. Thus, members of the Moissiacense group or species of viruses (i.e. Umbre virus strains), should be added to targeted tests in routine diagnosis of brain tissue from encephalitis cases with unidentified etiology, in particular in a context of immune suppression. The negativity of the CSF in such cases raises the question of brain biopsy at an early stage in the management of encephalitis of unknown etiology.

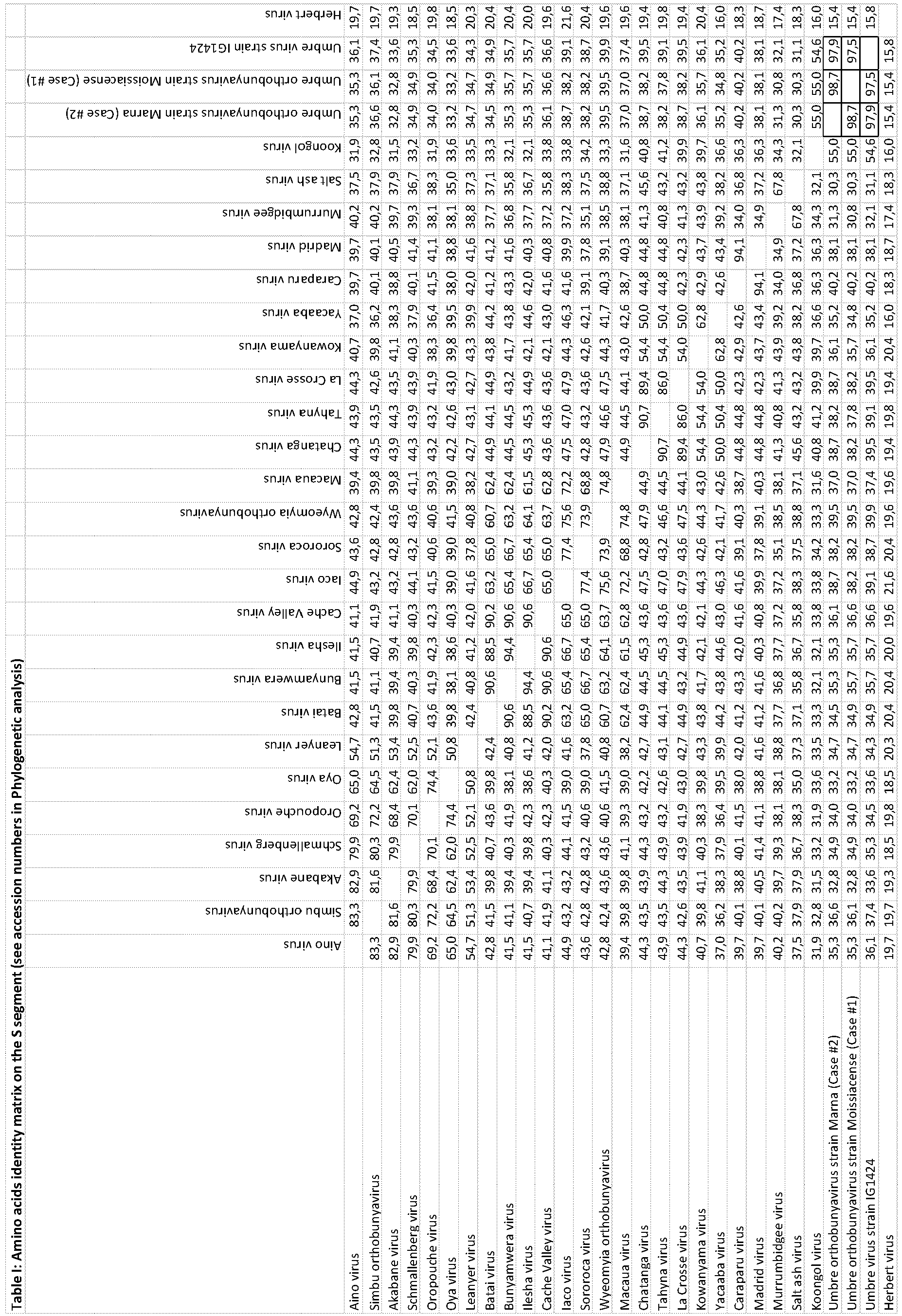

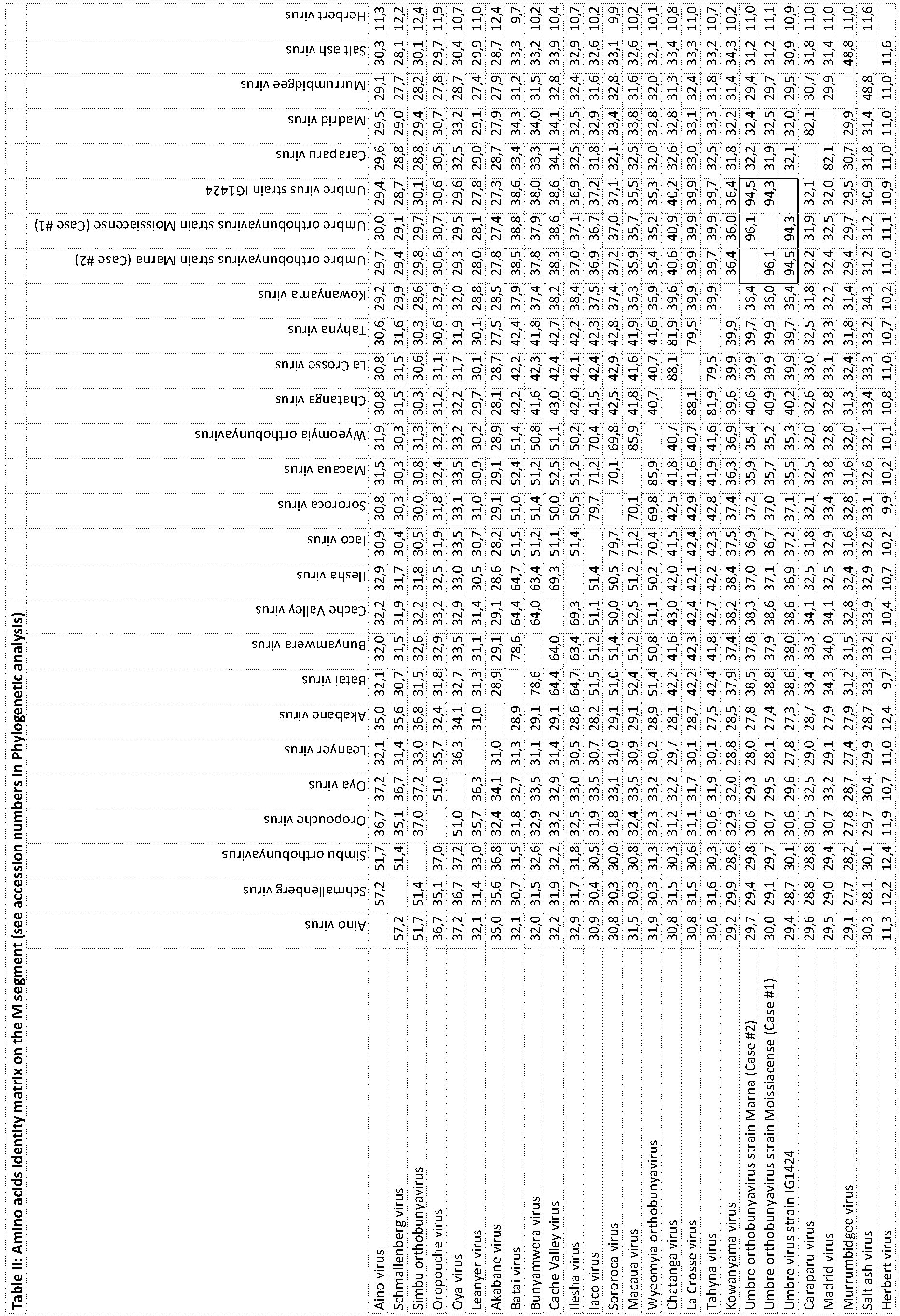

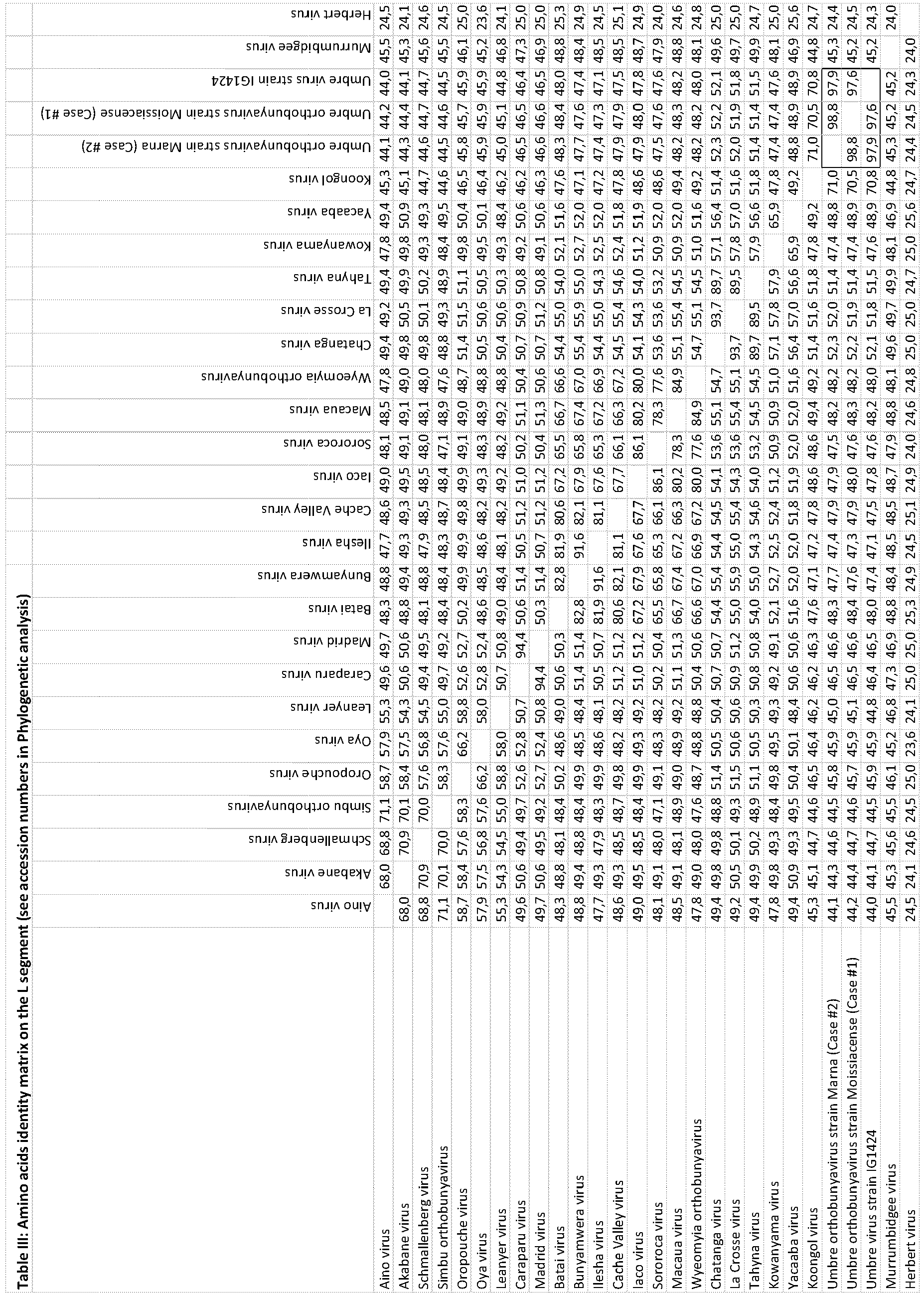

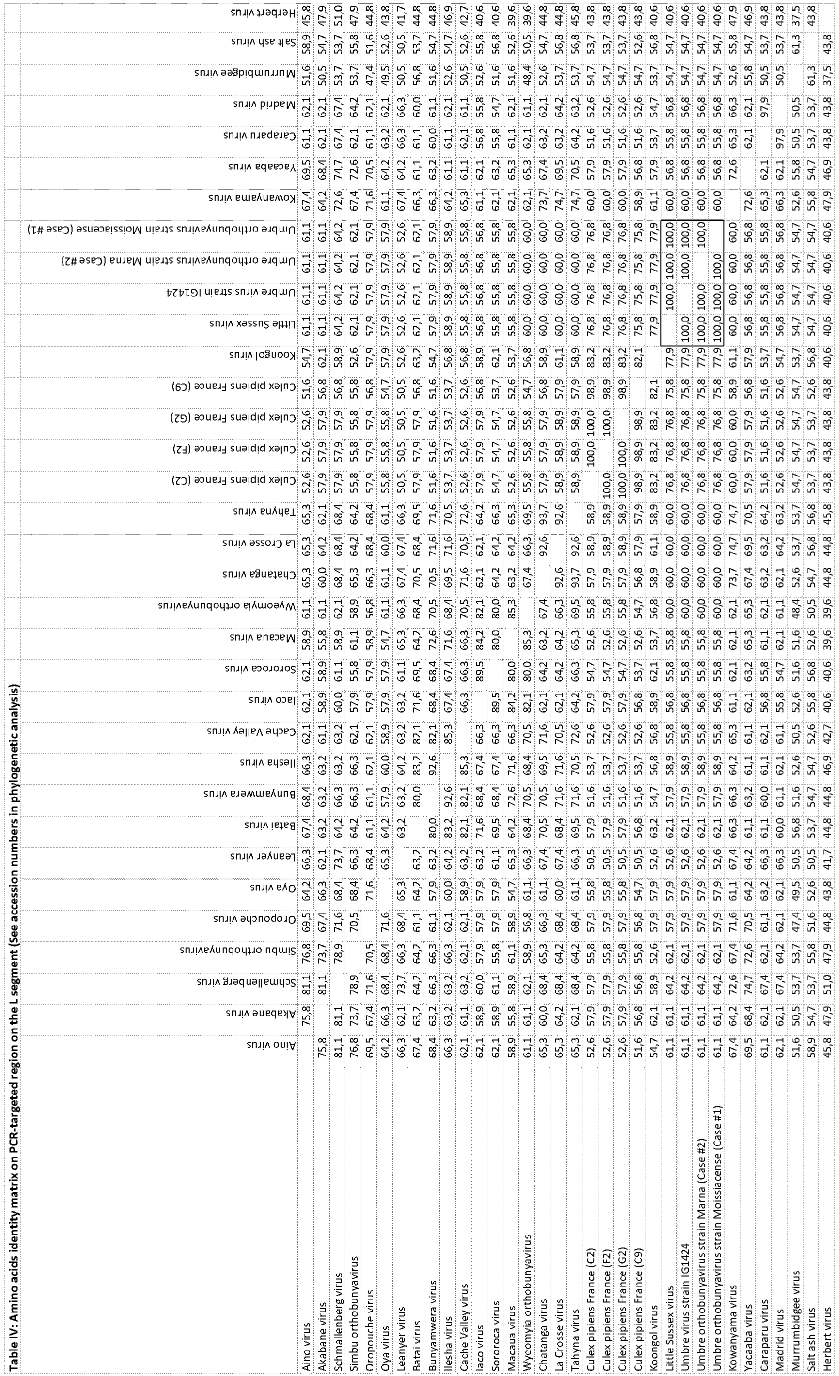

## Claims

1. A method of diagnosis of Moissiacense virus infection in a sample from an individual, comprising determining the presence of at least one viral nucleic acid selected from the group consisting of:
- a first nucleic acid (S segment) comprising a sequence having at least 80% identity with SEQ ID NO: 1 and further including a coding sequence for a Nucleocapsid (N) protein having at least 90% identity with SEQ ID NO: 2, or its complement;
- a second nucleic acid (M segment) comprising a sequence having at least 80% identity with SEQ ID NO: 3 and further including a coding sequence for a polyprotein M having at least 90% identity with SEQ ID NO: 4; or its complement; and
- a third nucleic acid (L segment) comprising a sequence having at least 80% identity with SEQ ID NO: 5 and further including a coding sequence for a polymerase (L) protein having at least 90% identity with SEQ ID NO: 6 including at least 90% identity on the core polymerase domain, which is from positions 954 to 1246 of SEQ ID NO: 6, or its complement.

2. The method according to claim 1, wherein said Moissiacense virus comprises: a S segment sequence of SEQ ID NO: 1 coding for a nucleocapsid (N) protein of SEQ ID NO: 2, a M segment sequence of SEQ ID NO: 3 coding for a polyprotein M of SEQ ID NO: 4 and a L segment sequence of SEQ ID NO: 5 coding for a polymerase (L) protein of SEQ ID NO: 6, or a S segment sequence of SEQ ID NO: 90 coding for a nucleocapsid (N) protein of SEQ ID NO: 93, a M segment sequence of SEQ ID NO: 91 coding for a polyprotein M of SEQ ID NO: 95, and a L segment sequence of SEQ ID NO: 92 coding for a polymerase (L) protein of SEQ ID NO: 96.

3. The method according to claim 1 or 2, which comprises the detection of at least one specific target sequence of the viral nucleic acid by a process of hybridization with an oligonucleotide probe, amplification with a pair of oligonucleotide primers, sequencing, or a combination thereof.

4. The method according to claim 3, wherein the at least one target sequence is chosen from the sequences SEQ ID NO: 7, 8, 9; the sequences of at least 15 nucleotides from any one of SEQ ID NO: 7 to 9, and the sequences comprising any one of SEQ ID NO: 7 to 9 and up to 40 consecutive nucleotides of 5' and/or 3' flanking sequence from the S, M or L segment sequence.

5. The method according to claim 3 or 4, wherein the oligonucleotide probe or primers are selected from the group consisting of: the sequences of 15 to 100 nucleotides, preferably 15 to 60 nucleotides having 80% to 100% identity with any of SEQ ID NO: 1, 3, 5, and 7 to 9, which are specific for Moissiacense virus; and their complement.

6. The method according to claim 5, wherein the oligonucleotide probe is selected from the group consisting of the sequences SEQ ID NO: 10 to 57 or the pair of oligonucleotide primers is selected from the group consisting of: SEQ ID NO: 10-11, 12-13, 14-15, 16-17, 18-19, 20-21, 22-23, 24-25, 26-27, 28-29, 30-31, 32-33, 34-35, 36-37, 38-39, 40-41, 42-43, 44-45, 46-47, 48-49, 50-51, 52-53, 54-55, and 56-57.

7. A method of diagnosis of Moissiacense virus infection in a sample from an individual, comprising:
- incubating an antigen from Moissiacense virus or antibody thereto with the sample to form a mixture, wherein said antigen comprises an amino acid sequence selected from the group consisting of: a sequence having 90 to 100% identity with SEQ ID NO: 2, SEQ ID NO: 58 or SEQ ID NO: 60 or a fragment of at least 10 amino acids of said sequence which is bound by antibodies against Moissiacense virus; and
- detecting antigen-antibody complexes in the mixture.

8. The method according to claim 7, wherein said fragment is an extracellular fragment from the sequence SEQ ID NO: 58, preferably chosen from the sequences SEQ ID NO: 61 to 64.

9. The method according to claim 7 or 8, which comprises an immunoprecipitation, ELISA or immunohistochemistry assay.

10. The method according to any one of claims 1 to 9, wherein the sample is brain biopsy, cerebral spinal fluid, whole-blood, plasma or serum.

11. The method according to any one of claims 1 to 10, wherein the same biological sample from the individual is subjected to a method of diagnosis of at least another encephalitis virus such as herpesvirus, enterovirus, polyomavirus, astrovirus, measles virus, mumps virus, or arbovirus.

12. The method according to any one of claims 1 to 11, which is for the diagnosis of encephalitis, in particular in humans, specifically for the differential diagnosis of human encephalitis.

13. A kit for the diagnosis or detection of Moissiacense virus, comprising at least one oligonucleotide probe or primer as defined in claim 5 or 6, an antigen as defined in claim 8 or an antibody thereto, preferably labelled, and eventually further comprising at least one oligonucleotide probe or primer, antigen or antibody thereto for the detection of another encephalitis virus such as herpesvirus, enterovirus, polyomavirus, astrovirus, measles virus, mumps virus, or arbovirus.

14. Use of the kit according to claim 13 for the detection of Moissiacense virus in arthropod vector population for epidemiological survey.

15. An immunogenic or vaccine pharmaceutical composition comprising, as active substance an antigen from Moissiacense virus as defined in claim 8, in association with at least one pharmaceutically acceptable vehicle, adjuvant and/or carrier.

## Patentansprüche

1. Verfahren zur Diagnose einer Moissiacense-Virusinfektion in einer Probe eines Individuums, umfassend das Bestimmen des Vorhandenseins von wenigstens einer viralen Nukleinsäure, ausgewählt aus der Gruppe gebildet aus:
- einer ersten Nukleinsäure (S-Segment), umfassend eine Sequenz mit wenigstens 80 % Identität mit der SEQ ID Nr. 1 und ferner einer kodierenden Sequenz für ein Nukleokapsid (N)-Protein mit wenigstens 90 % Identität mit der SEQ ID Nr. 2, oder seinem Komplement;
- einer zweiten Nukleinsäure (M-Segment), umfassend eine Sequenz mit wenigstens 80 % Identität mit der SEQ ID Nr. 3 und ferner umfassend eine kodierende Sequenz für ein Polyprotein M mit wenigstens 90 % Identität mit der SEQ ID Nr. 4; oder seinem Komplement; und
- einer dritten Nukleinsäure (L-Segment), umfassend eine Sequenz mit wenigstens 80 % Identität mit der SEQ ID Nr. 5 und ferner umfassend eine kodierende Sequenz für ein Polymerase (L)-Protein mit wenigstens 90 % Identität mit der SEQ ID Nr. 6, umfassend wenigstens 90 % Identität auf der Kernpolymerasedomäne, die sich von Position 954 bis 1246 von der SEQ ID Nr.6 erstreckt, oder ihrem Komplement.

2. Verfahren nach Anspruch 1, wobei das Moissiacense-Virus umfasst: eine S-Segment-Sequenz der SEQ ID Nr. 1, die für ein Nukleokapsid (N)-Protein der SEQ ID Nr. 2 kodiert, eine M-Segment-Sequenz der SEQ-ID Nr. 3, die für ein Polyprotein M der SEQ ID Nr. 4 kodiert, und eine L-Segment-Sequenz der SEQ-ID Nr. 5, die für ein Polymerase (L)-Protein der SEQ ID Nr. 6 kodiert, oder eine S-Segment-Sequenz der SEQ-ID Nr. 90, die für ein Nukleokapsid (N)-Protein der SEQ ID Nr. 93 kodiert, eine M-Segment-Sequenz der SEQ-ID Nr. 91, die für ein Polyprotein M der SEQ ID Nr. 95 kodiert, und eine L-Segment-Sequenz der SEQ-ID Nr. 92, die für ein Polymerase (L)-Protein der SEQ ID Nr. 96 kodiert.

3. Verfahren nach Anspruch 1 oder 2, das den Nachweis wenigstens einer spezifischen Zielsequenz der viralen Nukleinsäure durch ein Verfahren der Hybridisierung mit einer Oligonukleotid-Sonde, der Amplifikation mit einem Paar von Oligonukleotid-Primern, der Sequenzierung oder einer Kombination davon umfasst.

4. Verfahren nach Anspruch 3, wobei die wenigstens eine Zielsequenz ausgewählt ist aus den Sequenzen SEQ ID Nr. 7, 8, 9; den Sequenzen von wenigstens 15 Nukleotiden aus einer der SEQ ID Nr. 7 bis 9, und die Sequenzen, die eine der SEQ ID Nr. 7 bis 9 und bis zu 40 aufeinanderfolgende Nukleotide der 5'- und/oder 3'-flankierende Sequenz der S-, M- oder L-Segment-Sequenz umfassen.

5. Verfahren nach Anspruch 3 oder 4, wobei die Oligonukleotid-Sonde oder Primer ausgewählt sind aus der Gruppe, gebildet aus: den Sequenzen von 15 bis 100 Nukleotiden, bevorzugt 15 bis 60 Nukleotiden, die 80 % bis 100 % Identität mit irgendeiner der SEQ ID Nr. 1, 3, 5 und 7 bis 9 aufweisen, die spezifisch für das Moissiacense-Virus sind, und ihr Komplement.

6. Verfahren nach Anspruch 5, wobei die Oligonukleotidsonde ausgewählt ist aus der Gruppe, gebildet aus den Sequenzen SEQ ID Nr. 10 bis 57 oder das Oligonukleotid-Primerpaar ausgewählt ist aus der Gruppe, gebildet aus: SEQ-ID Nr.: 10-11, 12-13, 14-15, 16-17, 18-19, 20-21, 22-23, 24-25, 26-27, 28-29, 30-31, 32-33, 34-35, 36-37, 38-39, 40-41, 42-43, 44-45, 46-47, 48-49, 50-51, 52-53, 54-55 und 56-57.

7. Verfahren zur Diagnose einer Moissiacense-Virusinfektion in einer Probe eines Individuums, umfassend:
- Inkubieren eines Antigens aus dem Moissiacense-Virus oder eines Antikörpers dagegen mit der Probe, um ein Gemisch zu bilden, wobei das Antigen eine Aminosäuresequenz umfasst, die ausgewählt ist aus der Gruppe, gebildet aus: einer Sequenz mit 90 bis 100 % Identität mit SEQ ID Nr. 2, SEQ-ID NO.: 58, SEQ-ID NO.: 60 oder einem Fragment von wenigstens 10 Aminosäuren der genannten Sequenz, das von Antikörpern gegen das Moissiacense-Virus gebunden wird, und
- Nachweisen von Antigen-Antikörper-Komplexen in der Mischung.

8. Verfahren nach Anspruch 7, wobei das Fragment ein extrazelluläres Fragment der Sequenz SEQ ID NO. 58 ist, bevorzugt ausgewählt aus den Sequenzen SEQ ID Nr. 61 bis 64.

9. Verfahren nach Anspruch 7 oder 8, das eine Immunpräzipitation, einen ELISA oder einen immunhistochemischen Test umfasst.

10. Verfahren nach einem der Ansprüche 1 bis 9, wobei es sich bei der Probe um eine Hirnbiopsie, zerebrale Rückenmarksflüssigkeit, Vollblut, Plasma oder Serum handelt.

11. Verfahren nach einem der Ansprüche 1 bis 10, wobei dieselbe biologische Probe des Individuums einem Diagnoseverfahren für wenigstens ein anderes Enzephalitisvirus wie Herpesvirus, Enterovirus, Polyomavirus, Astrovirus, Masernvirus, Mumpsvirus oder Arbovirus unterzogen wird.

12. Verfahren nach einem der Ansprüche 1 bis 11 zur Diagnose von Enzephalitis, insbesondere beim Menschen, insbesondere zur Differentialdiagnose der menschlichen Enzephalitis.

13. Kit für die Diagnose oder den Nachweis von Moissiacense-Virus, umfassend wenigstens eine Oligonukleotid-Sonde oder einen Primer nach Anspruch 5 oder 6, ein Antigen gemäß Anspruch 8 oder einen Antikörper dagegen, bevorzugt markiert, und gegebenenfalls weiter umfassend wenigstens eine Oligonukleotid-Sonde oder einen Primer, ein Antigen oder einen Antikörper dagegen für den Nachweis eines anderen Enzephalitis-Virus wie Herpesvirus, Enterovirus, Polyomavirus, Astrovirus, Masernvirus, Mumpsvirus oder Arbovirus.

14. Verwendung des Kits nach Anspruch 13 zum Nachweis des Moissiacense-Virus in Arthropoden-Vektor-Populationen für epidemiologische Untersuchungen.

15. Pharmazeutische immunogene oder Impfstoff-Zusammensetzung, umfassend als Wirkstoff ein Antigen aus dem Moissiacense-Virus nach Anspruch 8 in Verbindung mit wenigstens einem pharmazeutisch akzeptablen Vehikel, Adjuvans und/oder Träger.

## Revendications

1. Méthode de diagnostic d'une infection par le virus Moissiacense dans un échantillon provenant d'un individu, comprenant la détermination de la présence d'au moins un acide nucléique viral choisi dans le groupe consistant en :
- un premier acide nucléique (segment S) comprenant une séquence ayant au moins 80 % d'identité avec SEQ ID NO: 1 et incluant, en outre, une séquence codante pour une protéine de nucléocapside (N) ayant au moins 90 % d'identité avec SEQ ID NO: 2, ou son complément ;
- un deuxième acide nucléique (segment M) comprenant une séquence ayant au moins 80 % d'identité avec SEQ ID NO: 3 et incluant, en outre, une séquence codante pour une polyprotéine M ayant au moins 90 % d'identité avec SEQ ID NO: 4 ; ou son complément ; et
- un troisième acide nucléique (segment L) comprenant une séquence ayant au moins 80 % d'identité avec SEQ ID NO: 5 et incluant en outre une séquence codante pour une protéine polymérase (L) ayant au moins 90% d'identité avec SEQ ID NO: 6 incluant au moins 90 % d'identité par rapport au domaine central de la polymérase, qui est compris entre les positions 954 et 1246 de SEQ ID NO:6, ou son complément.

2. Méthode selon la revendication 1, dans laquelle ledit virus Moissiacense comprend : une séquence de segment S de SEQ ID NO: 1 codant pour une protéine de nucléocapside (N) de SEQ ID NO: 2, un segment M de séquence SEQ ID NO: 3 codant pour une polyprotéine M de SEQ ID NO: 4, et un segment L de séquence SEQ ID NO: 5 codant pour une protéine de polymérase (L) de SEQ ID NO: 6, ou un segment S de séquence SEQ ID NO: 90 codant pour une protéine de nucléocapside (N) de SEQ ID NO: 93, un segment M de séquence SEQ ID NO: 91 codant pour une polyprotéine M de SEQ ID NO: 95, et un segment L de séquence SEQ ID NO: 92 codant pour une protéine de polymérase (L) de SEQ ID NO: 96.

3. Méthode selon la revendication 1 ou 2, qui comprend la détection d'au moins une séquence cible spécifique de l'acide nucléique viral par un processus d'hybridation avec une sonde oligonucléotidique, d'amplification avec une paire d'amorces oligonucléotidiques, de séquençage, ou une combinaison de ceux-ci.

4. Méthode selon la revendication 3, dans laquelle la au moins une séquence cible est choisie dans les séquences SEQ ID NO: 7, 8, 9 ; les séquences d'au moins 15 nucléotides de l'une quelconque des séquences SEQ ID NO: 7 à 9, et les séquences comprenant l'une quelconque des séquences SEQ ID NO: 7 à 9 et jusqu'à 40 nucléotides consécutifs de la séquence flanquante 5' et/ou 3' de la séquence du segment S, M ou L.

5. Méthode selon la revendication 3 ou 4, dans laquelle la sonde ou les amorces oligonucléotidiques sont choisies dans le groupe consistant en séquences de 15 à 100 nucléotides, de préférence, 15 à 60 nucléotides ayant 80 % à 100 % d'identité avec l'une quelconque des SEQ ID NO: 1, 3, 5 et 7 à 9, qui sont spécifiques du virus Moissiacense ; et leur complément.

6. Méthode selon la revendication 5, dans laquelle la sonde oligonucléotidique est choisie dans le groupe consistant en séquences SEQ ID NO: 10 à 57 ou la paire d'amorces oligonucléotidiques est choisie dans le groupe consistant en : SEQ ID NO: 10-11, 12-13, 14-15, 16-17, 18-19, 20-21, 22-23, 24-25, 26-27, 28-29, 30-31, 32-33, 34-35, 36-37, 38-39, 40-41, 42-43, 44-45, 46-47, 48-49, 50-51, 52-53, 54-55, et 56-57.

7. Méthode de diagnostic de l'infection par le virus Moissiacense dans un échantillon prélevé sur un individu, comprenant :
- l'incubation d'un antigène de virus Moissiacense ou d'un anticorps de celui-ci avec l'échantillon pour former un mélange, dans lequel ledit antigène comprend une séquence d'acides aminés choisie dans le groupe consistant en une séquence ayant 90 à 100 % d'identité avec SEQ ID NO: 2, SEQ ID NO: 58 ou SEQ ID NO: 60 ou un fragment d'au moins 10 acides aminés de ladite séquence qui est lié par des anticorps contre le virus Moissiacense ; et
- la détection de complexes antigène-anticorps dans le mélange.

8. Méthode selon la revendication 7, dans laquelle ledit fragment est un fragment extracellulaire de la séquence SEQ ID NO: 58,de préférence choisi dans les séquences SEQ ID NO: 61 à 64.

9. Méthode selon la revendication 7 ou 8, qui comprend un test d'immunoprécipitation, ELISA ou d'immunohistochimie.

10. Méthode selon l'une quelconque des revendications 1 à 9, dans laquelle l'échantillon est une biopsie cérébrale, du liquide céphalo-rachidien, du sang total, du plasma ou du sérum.

11. Méthode selon l'une quelconque des revendications 1 à 10, dans laquelle le même échantillon biologique de l'individu est soumis à une méthode de diagnostic d'au moins un autre virus de l'encéphalite tel qu'un herpèsvirus, un entérovirus, un polyomavirus, un astrovirus, un virus de la rougeole, un virus des oreillons ou un arbovirus.

12. Méthode selon l'une quelconque des revendications 1 à 11, qui est destinée au diagnostic de l'encéphalite, en particulier chez l'homme, notamment au diagnostic différentiel de l'encéphalite humaine.

13. Kit pour le diagnostic ou la détection du virus Moissiacense, comprenant au moins une sonde ou une amorce oligonucléotidique telle que définie dans la revendication 5 ou 6, un antigène tel que défini dans la revendication 8 ou un anticorps de celui-ci, de préférence marqué, et comprenant éventuellement, en outre, au moins une sonde ou une amorce oligonucléotidique, un antigène ou un anticorps de celui-ci pour la détection d'un autre virus de l'encéphalite tel qu'un herpèsvirus, un entérovirus, un polyomavirus, un astrovirus, un virus de la rougeole, un virus des oreillons, ou un arbovirus.

14. Utilisation du kit selon la revendication 13 pour la détection du virus Moissiacense dans la population d'arthropodes vecteurs pour une étude épidémiologique.

15. Composition pharmaceutique immunogène ou vaccinale comprenant, en tant que substance active, un antigène du virus Moissiacense tel que défini dans la revendication 8, en association avec au moins un véhicule, un adjuvant et/ou un support pharmaceutiquement acceptable.
